# EUROPEAN PATENT APPLICATION

(11) **EP 4 644 304 A1**
(43) Date of publication of application: **05.11.2025**
(21) Application number: 25172212.0
(22) Date of filing: 24.04.2025
(51) Int. Cl.: B65H 19/18

(54) **SYSTEM AND METHOD FOR FORMING A TAPELESS SPLICE BOND**

(30) Priority: 26.04.2024 US 202463639005 P; 29.07.2024 US 202463676572 P
(71) Applicant: The Procter & Gamble Company, Cincinnati, OH 45202 (US)
(72) Inventor: ELSHARAWY, Mohamed, 65824 Schwalbach am Taunus (DE); SCHNEIDER, Uwe, Cincinnati, 45202 (US); HAMDANI, Yogi, 53881 Euskirchen (DE)
(74) Representative: P&G Patent Germany

(57) **Abstract**

A method is presented for providing a continuous web (120, 130) to an absorbent article manufacturing line (149). The method includes conveying a first roll of material through a splice box (102). The method also includes providing a second roll of the material extending into the splice box. The method also includes sensing upcoming expiration of the first roll of material. The method also includes using a first and second vacuum to hold a respective first portion (133) of the first roll and a second portion (143) of the second roll against first and second components of a thermal welding apparatus. The method also includes forming a thermal weld at the splice location between the first and second portions (133, 143). The method also includes cutting the first roll of material upstream of the thermal weld. The method also includes conveying the second roll of material through the splice box (102) without stoppage of the absorbent article manufacturing line (149).

## Description

### CROSS REFERENCE TO RELATED APPLICATIONS

This application claims the benefit of U.S. Provisional Application No. 63/639,005, filed April 26, 2024, and U.S. Provisional Application No. 63/676,572, filed July 29, 2024, the entire disclosures of which are fully incorporated by reference herein.

### FIELD

The present disclosure relates generally to forming a splice bond between an expiring roll of web and a new roll of web on an absorbent article manufacturing line and more specifically to a system and method for forming a tapeless splice bond between the expiring roll of web and a new roll of web on the absorbent article manufacturing line.

### BACKGROUND

A splice box is a machine element and assembly that is responsible for the task of connecting running material of an expiring roll with a static material of a new roll, and by that creating a continuous non-interrupted production line process on a web converting line.

Conventional splice boxes have drawbacks including requiring tape to form a splice between the material of the expiring roll and the material of the new roll which has limited strength and requires manual labor of offline preparation. Consequently, these conventional splice boxes typically require that an increased amount of material from the expiring roll be accumulated prior to the splicing process. Additionally, some conventional splice boxes form butt splices between the material of the expiring roll and the material of the new roll which also have limited strength. Furthermore, conventional splice boxes have limited compatibility with robotic automation and thus usually require that several steps of the process be manually performed by an operator, thereby reducing the overall efficiency of the process.

The discussion of shortcomings and needs existing in the field prior to the present disclosure is in no way an admission that such shortcomings and needs were recognized by those skilled in the art prior to the present disclosure.

### SUMMARY

Various embodiments solve the above-mentioned problems and provide methods and devices useful for forming a tapeless splice bond between material of an expiring roll and material of a new roll on an absorbent article manufacturing line. Additionally, various embodiments solve the above-mentioned problems by providing methods and devices for forming an overlapping splice bond between the material of the expiring roll and the material of the new roll, thereby forming a stronger splice bond than conventional methods. Yet further, various embodiments herein provide methods and devices that are compatible with robotic automation for many or all of the process steps. Still further, various embodiments herein provide methods and devices that remove damaged outer layers of the new roll material prior to the splicing with the material of the expiring roll. Additionally, various embodiments herein provide methods and devices that are capable of forming the tapeless splice bond between the material of the expiring roll and the material of the new roll without any interruption to the absorbent article manufacturing line.

The embodiments herein discuss a method or apparatus for joining material of an expiring web to material of a new web, where the webs overlap each other under tension before they are simultaneously fused and cut. In some embodiments, the fusion and cutting action occurs by a thermal bonding apparatus comprising a knife cutting system and an ultrasonic system comprised of a Sonotrode (horn) and an anvil. Additionally, in these embodiments, the thermal bonding apparatus bonds across the full width of the overlapped web, either a continuous bond or an intermittent one.

In a first set of embodiments, a method is presented for providing a continuous web to an absorbent article manufacturing line. The method includes the step of conveying a first portion of a first roll of material through a splice box and into the absorbent article manufacturing line including accumulating the first roll of material downstream of a splice location in the splice box. The method also includes that prior to expiration of the first roll of material, a step of providing a second roll of the material extending into the splice box. The method also includes the step of sensing an upcoming expiration of the first roll of material. The method also includes using a vacuum to hold a second portion of the second roll of the material proximate to a leading edge thereof against a second component of a thermal welding apparatus. The method also includes a step of sensing an upcoming expiration of the first roll of material. The method also includes positioning a first component proximate to the second component of the thermal welding apparatus. The method also includes positioning the first portion of the first roll in an overlapping configuration with the second portion of the second roll. The method also includes forming a thermal weld or splice bond at the splice location between the first portion of the first roll of material and the second portion of the second roll of material using the thermal welding apparatus. The first portion of the first roll comprises, in a cross-machine direction, a first side region and a second side region separated by a central region. The second portion of the second roll comprises, in the cross-machine direction, a first side region and a second side region separated by a central region. The thermal weld is formed in the first side regions, the central regions, and the second side regions of the first and second rolls at the same time. The method also includes the step of cutting the first roll of material upstream of the thermal weld. The method also includes the step of conveying the second roll of material through the splice box and into the absorbent article manufacturing line without stoppage of the absorbent article manufacturing line.

In a second set of embodiments, a method is presented for providing a continuous web to an absorbent article manufacturing line. The method includes the step of conveying a first roll of material through a splice box and into the absorbent article manufacturing line. The method also includes the step of providing a second roll of the material extending into the splice box, prior to expiration of the first roll of material. The method also includes the step of extending the second roll of material into the splice box to a position downstream of a splice location. The method also includes the step of collecting a portion of the second roll of material downstream of the splice location to remove a plurality of outer layers of the second roll of material. The method also includes the step of cutting the collected material from a remainder of the second roll thereby forming the leading edge in the second roll of material. The method also includes the step of retracting the leading edge of the second roll of material into the splice location to reduce or eliminate a first tail being formed in the continuous web. The method also includes positioning a first portion of the first roll of the material proximate to a tailing edge thereof adjacent a first component of a thermal welding apparatus at the splice location. The method also includes using a vacuum to hold a second portion of the second roll of the material proximate to the leading edge thereof against a second component of the thermal welding apparatus at the splice location. The method also includes positioning the first component of the thermal welding apparatus proximate to the second component of the thermal welding apparatus. The method also includes positioning the first portion of the first roll in an overlapping configuration with the second portion of the second roll. The method also includes the step of forming a thermal weld or splice bond at the splice location between the first portion of the first roll of material and the second portion of the second roll of material using the first and second components of the thermal welding apparatus. The first portion of the first roll comprises, in a cross-machine direction, a first side region and a second side region separated by a central region. The second portion of the second roll comprises, in the cross-machine direction, a first side region and a second side region separated by a central region. The thermal weld is formed in the first side regions, the central regions, and the second side regions of the first and second rolls at the same time. The method also includes cutting the first roll of material upstream of the thermal weld. The method also includes conveying the second roll of material through the splice box and into the absorbent article manufacturing line without stoppage of the absorbent article manufacturing line.

These and other features, aspects, and advantages of various embodiments will become better understood with reference to the following description, figures, and claims.

### BRIEF DESCRIPTION OF THE DRAWINGS

Many aspects of this disclosure can be better understood with reference to the following figures, which illustrate examples according to various embodiments.
FIG. 1A is a block diagram that illustrates an example of a system for forming a tapeless splice bond between material of an expiring roll and a new roll, according to various embodiments;
FIG. 1B is a block diagram that illustrates an example of the system of FIG. 1A upon detection of an upcoming expiration of the expiring roll, according to various embodiments;
FIG. 1C is a block diagram that illustrates an example of the system of FIG. 1A upon fixing a first portion of the expiring roll adjacent the sonotrode and a second portion of the new roll adjacent the anvil, according to various embodiments;
FIG. 1D is a block diagram that illustrates an example of the system of FIG. 1A upon forming a splice bond between the first portion of the expiring roll and the second portion of the new roll, according to various embodiments;
FIG. 2A is a block diagram that illustrates an example of the system of FIG. 1A where outer layers of the new roll are collected downstream of the splice location, according to various embodiments;
FIG. 2B is a block diagram that illustrates an example of the system of FIG. 1A where the second portion of the new roll is fixed adjacent the anvil, according to various embodiments;
FIG. 2C is a block diagram that illustrates an example of the system of FIG. 1A where the collected outer layers are cut from the second roll, according to various embodiments;
FIG. 3A is a side view that illustrates an example of the sonotrode and anvil of the system of FIG. 1C forming the splice bond, according to various embodiments;
FIG. 3B is a perspective view that illustrates an example of the sonotrode and anvil of the system of FIG. 1C forming the splice bond, according to various embodiments;
FIG. 3C is a side view that illustrates an example of the splice bond formed between material of the expiring and new rolls in the system of FIG. 1D, according to various embodiments;
FIG. 3D is a front view that illustrates an example of the splice bond formed between materials of the expiring and new rolls in the system of FIG. 1D, according to various embodiments;
FIGS. 4A and 4B are front views that illustrates an example of the anvil and adjacent sensors to detect a presence of the leading edge of the new roll in the system of FIG. 1B, according to various embodiments;
FIGS. 5A and 5B are side views that illustrates an example of the mandrel of the new roll and a vacuum device to automatically pick up the leading edge of the new roll, according to various embodiments;
FIGS. 5C and 5D are side views that illustrates an example of the mandrel of the new roll and an air blade and mechanical finger to automatically pick up the leading edge of the new roll, according to various embodiments;
FIG. 5E is a top view of a conventional sticker used to secure a leading edge of a new roll;
FIG. 5F is a top view that illustrates an example of a multi-zone sticker to secure the leading edge of the new roll and facilitate automated pick up of the leading edge of the new roll;
FIG. 5G is a cross-sectional view of the multi-zone sticker of FIG. 5F taken along the line 5G-5G;
FIG. 5H is a top view that illustrates an example of indicia provided on one or more zones of the multi-zone sticker of FIG. 5F;
FIG. 5I is a top perspective view that illustrates an example of a cantilevered splice box used in the system of FIG. 1A;
FIG. 5J is a top perspective view that illustrates an example of a non-cantilevered splice box used in the system of FIG. 1A;
FIG. 6 is a flowchart that illustrates an example of a method for forming a tapeless splice bond between material of the expiring roll and material of the new roll, according to various embodiments;
FIG. 7 is a block diagram that illustrates a computer system upon which an embodiment of the invention may be implemented;
FIG. 8 is a block diagram that illustrates a chip set upon which an embodiment of the invention may be implemented;
FIG. 9 is a block diagram that illustrates a mobile terminal upon which an embodiment of the invention may be implemented; and
FIG. 10 is a perspective view of a bundle of rolls of webs of material wrapped in a film and with a sacrificial material positioned intermediate the film and the webs.

It should be understood that the various embodiments are not limited to the examples illustrated in the figures.

### DETAILED DESCRIPTION

### Introduction and Definitions

This disclosure is written to describe the invention to a person having ordinary skill in the art, who will understand that this disclosure is not limited to the specific examples or embodiments described. The examples and embodiments are single instances of the invention which will make a much larger scope apparent to the person having ordinary skill in the art. Unless defined otherwise, all technical and scientific terms used herein have the same meaning as commonly understood by the person having ordinary skill in the art. It is also to be understood that the terminology used herein is for the purpose of describing examples and embodiments only, and is not intended to be limiting, since the scope of the present disclosure will be limited only by the appended claims.

All the features disclosed in this specification (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features. The examples and embodiments described herein are for illustrative purposes only and that various modifications or changes in light thereof will be suggested to the person having ordinary skill in the art and are to be included within the spirit and purview of this application. Many variations and modifications may be made to the embodiments of the disclosure without departing substantially from the spirit and principles of the disclosure. All such modifications and variations are intended to be included herein within the scope of this disclosure. For example, unless otherwise indicated, the present disclosure is not limited to particular materials, reagents, reaction materials, manufacturing processes, or the like, as such can vary. It is also to be understood that the terminology used herein is for purposes of describing particular embodiments only and is not intended to be limiting. It is also possible in the present disclosure that steps can be executed in different sequence where this is logically possible.

All numeric values are herein assumed to be modified by the term "about," whether or not explicitly indicated. The term "about" generally refers to a range of numbers that one of skill in the art would consider equivalent to the recited value (for example, having the same function or result). In many instances, the term "about" may include numbers that are rounded to the nearest significant figure.

In everyday usage, indefinite articles (like "a" or "an") precede countable nouns and noncountable nouns almost never take indefinite articles. It must be noted, therefore, that, as used in this specification and in the claims that follow, the singular forms "a," "an," and "the" include plural referents unless the context clearly dictates otherwise. Thus, for example, reference to "a support" includes a plurality of supports. Particularly when a single countable noun is listed as an element in a claim, this specification will generally use a phrase such as "a single." For example, "a single support."

Unless otherwise specified, all percentages indicating the amount of a component in a composition represent a percent by weight of the component based on the total weight of the composition. The term "mol percent" or "mole percent" generally refers to the percentage that the moles of a particular component are of the total moles that are in a mixture. The sum of the mole fractions for each component in a solution is equal to 1.

Where a range of values is provided, it is understood that each intervening value, to the tenth of the unit of the lower limit (unless the context clearly dictates otherwise), between the upper and lower limit of that range, and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

In this specification and in the claims that follow, reference will be made to a number of terms that shall be defined to have the following meanings unless a contrary intention is apparent.

"Absorbent article" refers to devices that absorb and contain liquid, and more specifically, refers to devices that are placed against or in proximity to the body of the wearer to absorb and to contain various exudates discharged from the body.

"Machine direction" (MD) refers to the direction of material flow through a process. In addition, relative placement and movement of material can be described as flowing in the machine direction through a process from upstream in the process to downstream in the process.

"Cross direction" (CD) refers to a direction that is generally perpendicular to the machine direction.

### System for Forming a Tapeless Splice Bond

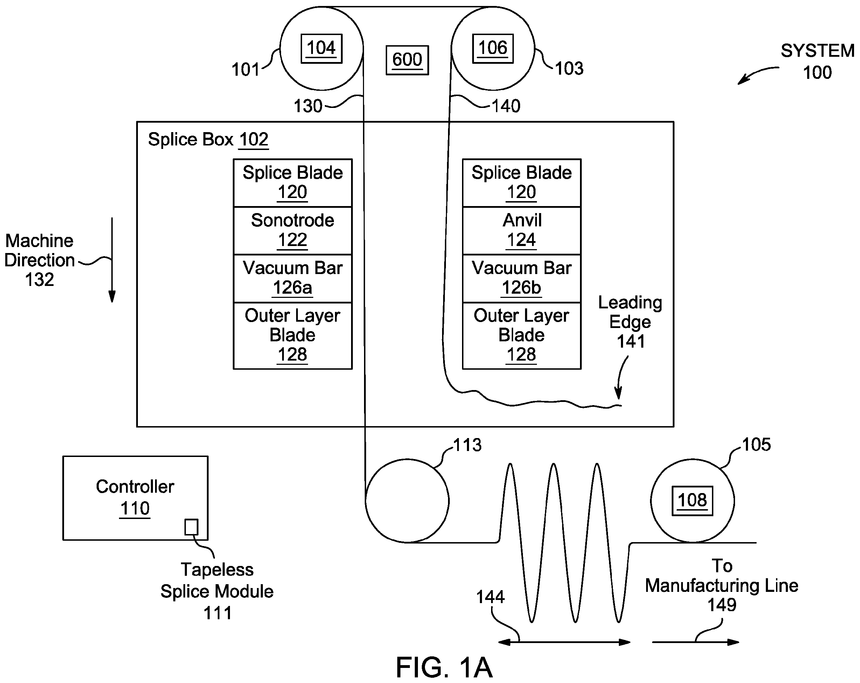

A system for forming a tapeless splice bond between an expiring roll of material and a new roll of material in an absorbent article manufacturing line will now be discussed. FIG. 1A is a block diagram that illustrates an example of a system 100 for forming a tapeless splice bond between material of an expiring roll and a new roll, according to various embodiments. In one embodiment, the material of the expiring roll is a first web or an expiring web 130 that has a tailing edge (not shown) and the material of new roll is a second web or new web 140 that has a leading edge 141.

Although some related art methods and devices are disclosed that form a tapeless splice bond between an expiring roll of material and a new roll of material, such as WO 2024/34461A1 to Zuiko ("Zuiko" hereafter), this related art method and device also features notable drawbacks. For example, Zuiko requires that the manufacturing line be shut down during the splicing process, which severely impacts the overall efficiency of the absorbent article manufacturing line. Yet further, Zuiko does not remove outer layers of the new roll of material which may need to be discarded (e.g. due to hygiene and/or routine wear or damage of outer layers of the new roll) prior to the splicing process. Hence, the method of Zuiko potentially forms a splice bond between the material of the expiring roll and damaged material of the outer layers of the new roll.

The system will now be discussed prior to initiating the splicing process. As shown in FIG. 1A, the expiring web 130 is conveyed from a first mandrel 101, through a splice box 102 in a machine direction (MD) 132, over an undriven roll 113 and to a second mandrel 105 to an absorbent article manufacturing line 149. In one example embodiment, the first mandrel 101 has a first motor 104 and the second mandrel 105 has a second motor 108. The first motor 104 is configured to rotate the first mandrel 101 in either a clockwise or counterclockwise direction. In one example embodiment, clockwise rotation of the first mandrel 101 and counterclockwise rotation of the undriven roll 113 causes the expiring web 130 to move through the splice box 102 to the manufacturing line 149. A controller 110 is provided that is communicatively coupled with the motor 104 to send signals to the motor 104 to adjust a magnitude or direction of the rotation speed of the mandrel 101 and thus adjust a magnitude or direction of the rotation speed of the expiring web 130 over the mandrel 101 and undriven roll 113.

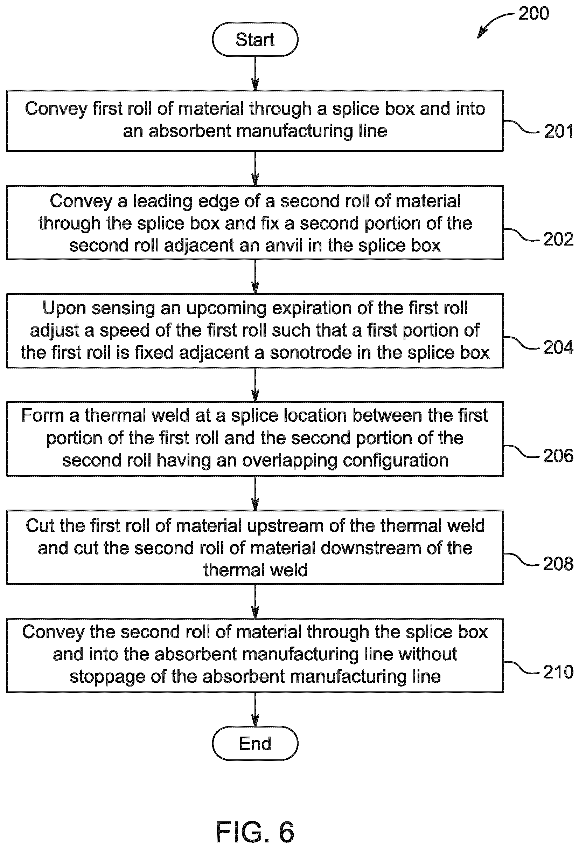

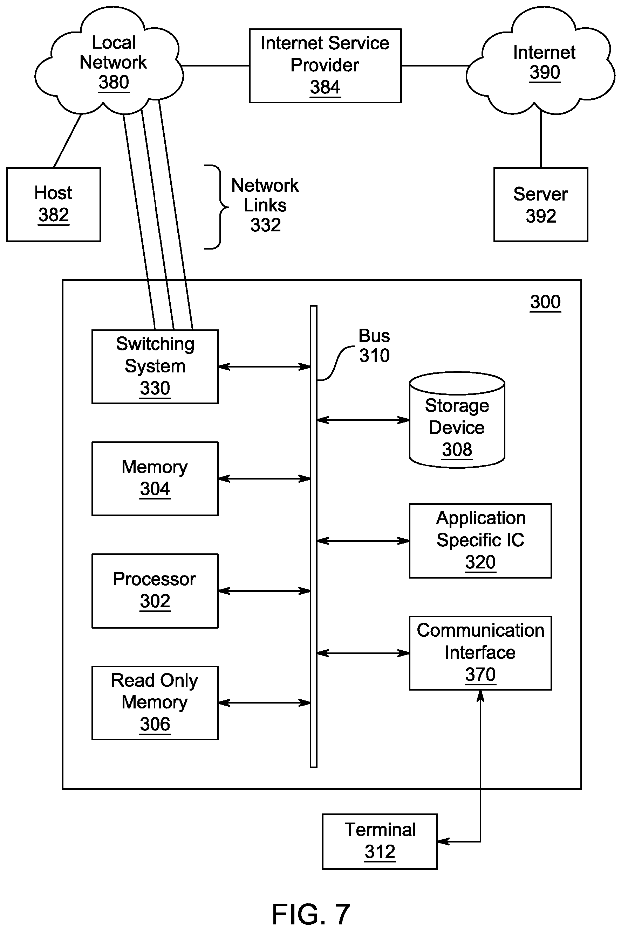

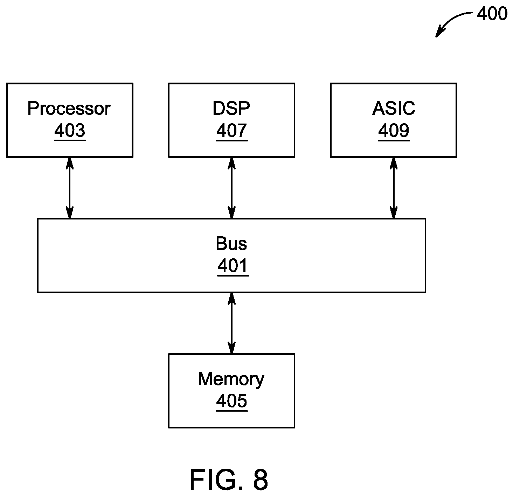

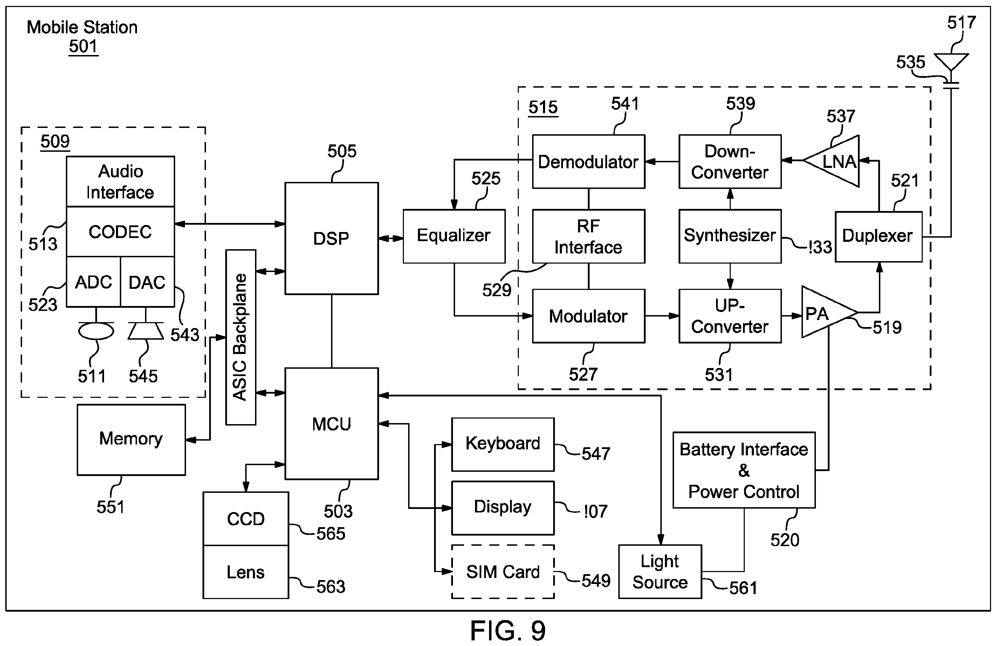

The controller 110 may include a passive generator and safety circuit 111. The controller 110 may perform one or more steps of the method 200 discussed with respect to the flowchart of FIG. 6. In some embodiments, the processor or controller 110 is a computer system as described below with reference to FIG. 7, a chip set described below with reference to FIG. 8 or a mobile terminal described below with reference to FIG. 9.

As further shown in FIG. 1A, in one example embodiment an accumulated portion or splicer buffer system 144 of the expiring web 130 is collected downstream of the splice box 102. In one embodiment, this splicer buffer system 144 is collected using mandrels that cause the expiring web 130 to form a serpentine path downstream of the splice box 102 and upstream of the absorbent article manufacturing line 149. This splicer buffer system 144 is used to continuously feed the expiring web 130 to the absorbent article manufacturing line 149 in the event that the expiring web 130 is slowed or stopped upstream of the splice box 102. This could occur for various reasons including the method disclosed herein where the expiring web 130 is slowed down and stopped to form a splice bond between the expiring and new web 130, 140. In some embodiments, once the splicing process herein is initiated the value of the splicer buffer system 144 can be increased such that by the time that the expiring web 130 is stopped to form the splice bond the splicer buffer system 144 has increased to a value that is sufficient to ensure no stoppage of the web material to the manufacturing line 149 during the process steps herein.

As further shown in FIG. 1A, in one embodiment the new web 140 is provided prior to expiration of the expiring web 130, where the new web 140 similarly extends into the splice box 102 in the MD direction 132. As shown in FIG. 1A, in this embodiment the new web 140 is conveyed from a third mandrel 103 to the splice box 102 in the MD direction 132. As with the other mandrel 101, the third mandrel 103 features a motor 106 which is communicatively coupled with the controller 110 which sends signals to the motor 106 to adjust a magnitude or direction of the rotation speed of the mandrel 103 and thus a magnitude or direction of the rotation speed of the new web 140 over the mandrel 103.

In some embodiments, again referring to FIG. 1A, a visual inspection device 600, such as a camera, may be positioned proximate to the mandrel 103. The visual inspection device 600 may be positioned at any suitable position proximate to the mandrel 103 so that it is configured to detect when one full defect-free revolution of the new web 140 is unwound. As such, instead of initially unwinding a certain number of revolutions (e.g., 10) from the new web 140, only the amount of damaged material may be unwound until a defect free revolution is detected. This can attribute to significant material savings by reducing scrap.

In some embodiments, the expiring web 130 and the new web 140 are the same material. In an example embodiment, the expiring web 130 and the new web 140 each include a nonwoven material, a film, or a laminate including a nonwoven material and a film. In still another example embodiment, the expiring web 130 or the new web 140 include a laminate of more than one material. In other embodiments, the expiring web 130 and new web 140 may be different material (e.g. bond cross of different material).

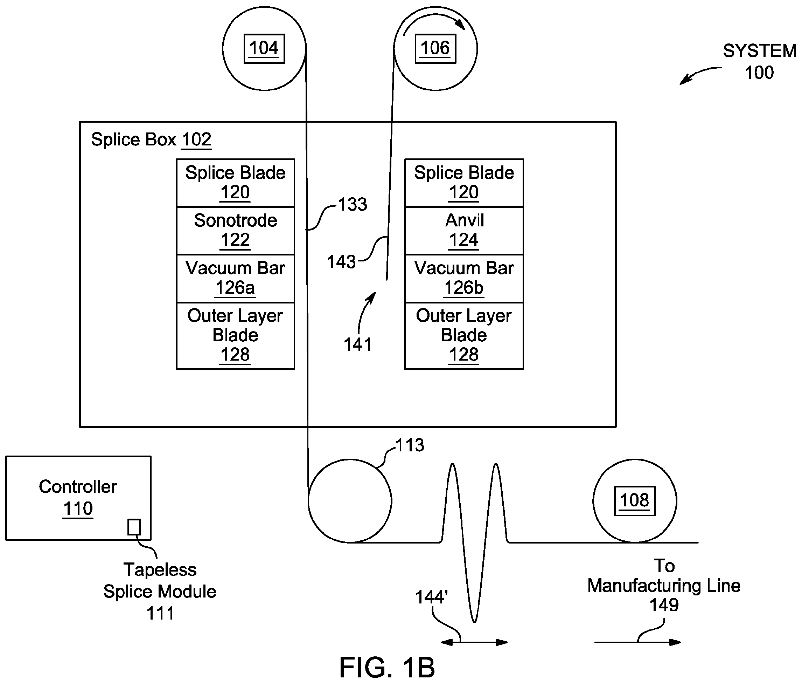

The control of the new web 140 will now be discussed. In some embodiments, the roll of the new web 140 includes outer layers which are to be removed prior to using the new web 140 in the slicing process disclosed herein. This is due to various reasons (e.g. hygiene) or conditions (e.g. puncture with a forklift) that can routinely cause damage to these outer layers prior to conveying the new web 140 into the splice box 102. In one embodiment, these outer layers are removed manually from the new web 140 prior to moving the leading edge 141 of the new web 140 into the splice box 102 or at least downstream of a splice location (e.g. location of the sonotrode 122 and anvil 124 within the splice box 102) as shown in FIG. 1A. As shown in FIG. 1B, the controller 110 transmits a signal to the motor 106 of the third mandrel 103 to cause the third mandrel 103 to rotate clockwise and thus move the leading edge 141 in an upstream direction (opposite to the MD direction 132). In this embodiment, the controller 110 sends a signal to the motor 106 to stop or slow down rotation of the third mandrel 103 upon detecting that the leading edge 141 is within a threshold distance of the second component (e.g. anvil 124) of the thermal bonding apparatus and/or that a second portion 143 of the new web 140 is adjacent to the second component of the thermal bonding apparatus. As discussed in later embodiments, a sensor (not shown) is positioned to detect the presence of the leading edge 141 within the threshold distance of the second component of the thermal bonding apparatus. This sensor is configured to transmit a signal to the controller 110 after which the controller 110 signals the motor 106 to cease or slow down rotation of the third mandrel 103. The new web 140 features a second clamping mechanism (not shown), such as the vacuum bar 126b, which holds the second portion 143 of the second web 140 against the second component (e.g. anvil 124) of the thermal bonding apparatus, prior to and during the splice bonding step. However, although FIG. 1B depicts that the second portion 143 is spaced apart from the anvil 124, this is for ease of illustration and the second portion 143 would be in contact against the anvil 124 as the leading edge 141 is retracted back in the upstream direction to within the threshold distance of the anvil 124.

An initial step of the splicing process is now discussed herein, where an upcoming expiration of the expiring web 130 is detected. FIG. 1B is a block diagram that illustrates an example of the system of FIG. 1A upon detection of an upcoming expiration of the expiring web 130, according to various embodiments. In some embodiments, a sensor (not shown) is provided to detect the upcoming expiration of the web 130 and transmit a signal to the controller 110 to indicate the upcoming expiration. In some embodiments, the sensor is on the mandrel 101 of the expiring web 130 (not shown) and detects when a number of remaining layers on the mandrel 101 is below a threshold number. This sensor can detect when a radial thickness of the remaining expiring web 130 on the mandrel 101 falls below a threshold value. In other embodiments, the sensor is an imaging device (e.g. camera) which detects that the radial thickness of the remaining expiring web 130 on the mandrel 101 is less than the threshold value.

The control of the expiring web 130 will now be discussed after detecting the upcoming expiration. Upon receiving the signal from the sensor indicating the upcoming expiration of the web 130, the controller 110 transmits one or more signals to the motor 104 of the mandrel 101 to slow down the expiring web 130 upstream of the splice box 102. In this embodiment, the controller 110 does not transmit a signal to the motor 108 of the mandrel 105 downstream of the splicer buffer system 144 and thus the speed of the expiring web 130 incident on the manufacturing line 149 remains unchanged. Thus, FIG. 1B depicts that the splicer buffer system 144' of the expiring web 130 has reduced from the splicer buffer system 144 of the expiring web 130 in FIG. 1A prior to detection of the upcoming expiration of the web 130. When the splice bond is to be formed between the webs 130, 140, the controller 101 transmits a signal to the motor 104 to cease rotation of the mandrel 101 such that a first portion 133 of the expiring web 130 is adjacent to a first component (e.g. sonotrode 122) of a thermal welding apparatus in the splice box 102. The thermal welding apparatus of the splice box 102 is used to form a splice bond between the expiring web 130 of the expiring roll and the new web 140 of the new roll.

Although the previously discussed embodiment teaches that the controller 101 transmits a signal to the motor 104 of the mandrel 101 to slow down the expiring web 130 upon detecting an upcoming expiration, in other embodiments the controller 101 may transmit a signal to the motor 104 of the mandrel 101 to speed up the expiring web 130 upon detecting the upcoming expiration. This may advantageously increase the splicer buffer system 144 from what is depicted in FIG. 1A (e.g. since the speed of the expiring web 130 incident on the manufacturing line 149 remains unchanged).

Although the previously discussed embodiment teaches that the splicer buffer system 144 of the expiring web 130 is collected downstream of the splice box 102, in other embodiments no splicer buffer system 144 of the expiring web 130 is collected downstream of the splice box 102. In this embodiment, once the upcoming expiration of the web 130 is detected by the sensor, the controller 110 transmits signals to the motor 104 so that the mandrel 101 ceases rotation when the first portion 133 of the expiring web 130 is adjacent to the sonotrode 122. In this embodiment, there is a stoppage in the expiring web 130 to the manufacturing line 149 during the splice process.

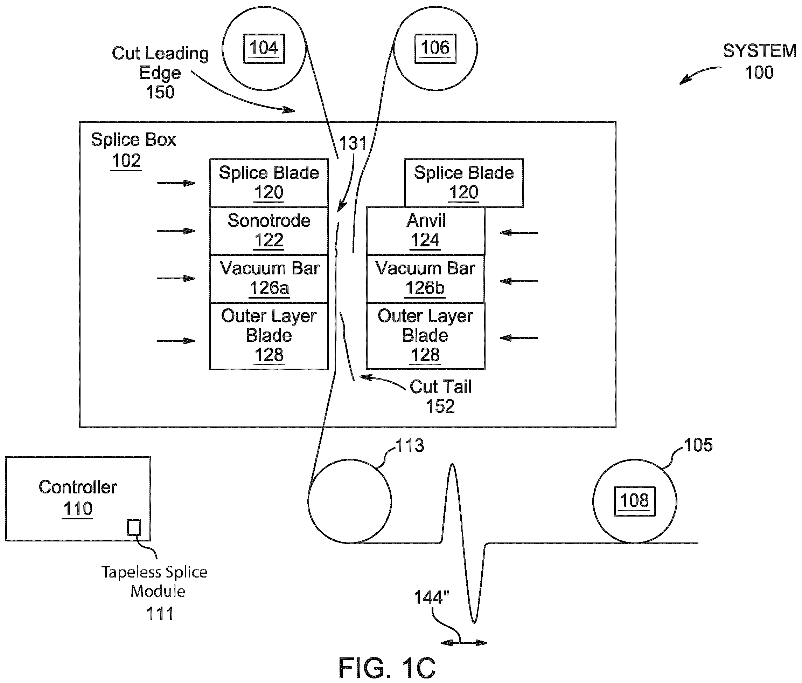

A second step of the splicing process will now be discussed, where the splice bond is formed between the expiring and new webs 130, 140 and excess tails of the expiring and new webs 130, 140 are cut away after forming the splice bond. FIG. 1C is a block diagram that illustrates an example of the system 100 of FIG. 1A upon fixing the first portion 133 of the expiring web 130 adjacent the sonotrode 122 and the second portion 143 of the new web 140 adjacent the anvil 124. In some embodiments, before the detection of the upcoming expiration of the web 130 and before the splice bond is formed between the expiring and new webs 130, 140, a vacuum of the vacuum bar 126b is used to hold the second portion 143 of the new web 140 against the anvil 124. In some embodiments, the vacuum is a fluid pressure and a portion of the fluid pressure is provided through the source of vibration energy. As shown in FIG. 1B, the first portion 133 of the expiring web 130 and the second portion 143 of the new web 140 are in an overlapping configuration in the MD direction 132. This is due to the expiring web 130 extending upstream of the splice location (location of the thermal bonding apparatus) whereas the leading edge 141 being positioned downstream of the splice location. The overlapping configuration of the first and second portions 133, 143 is preferred over a butt splice to survive the web converter up to the reject gate of the splice end of the line. In some embodiments, non-overlapping splices may be too weak, such that the web breaks at splice point and leads to web loss and effort/time lost on production line. Although FIG. 1B depicts the first and second portions 133, 143 spaced apart from the sonotrode 122 and anvil 124, this is for ease of illustration and in some embodiments the first portion 133 and second portion 143 would be in contact against the sonotrode 122 and anvil 124 during the splice bonding step.

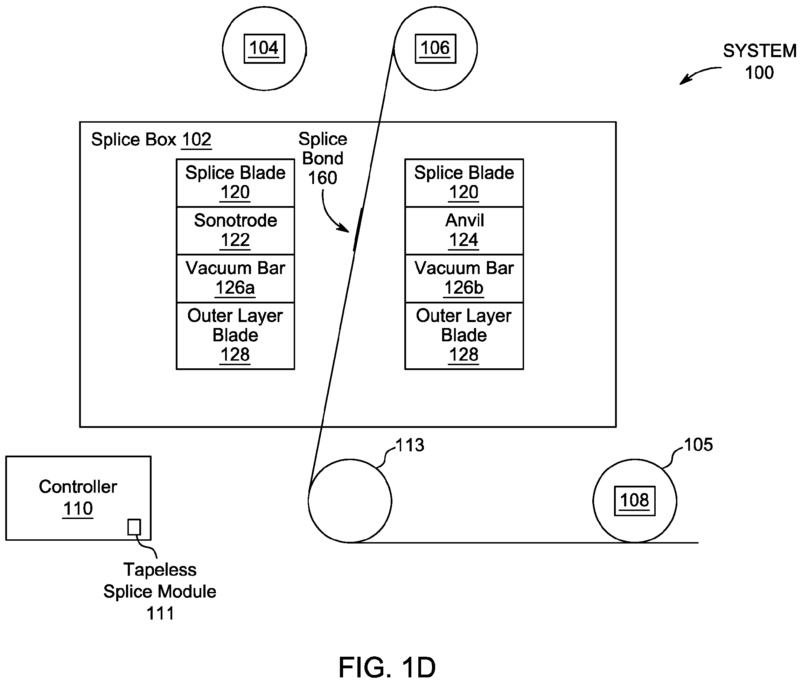

After fixing the first and second portions 133, 143 of the expiring and new webs 130, 140 against the respective first and second components of the thermal bonding apparatus, the first and second components of the thermal bonding apparatus are moved together to form the splice bond. As shown in FIG. 1C, in an embodiment upon sensing that the first and second portions 133, 143 are fixed against the first and second components of the thermal bonding apparatus, the controller 110 transmits a signal to a motor (not shown) to move the first and second components of the thermal bonding apparatus together. In this example embodiment, the motor causes the sonotrode 122 and anvil 124, as well as the vacuum bars 126a, 126b to move together in a direction orthogonal to the MD direction 132. This may be due to the vacuum bar 126a being on the same carriage as the sonotrode 122 and the vacuum bar 126b being on the same carriage as the anvil 124 (e.g. such that they move together with one pneumatic cylinder). The controller 110 then signals the sonotrode 122 and anvil 124 to activate and the splice bond 160 (FIG. 1D) is then formed between the first and second portions 133, 143 of the expiring and new webs 130, 140.

In some embodiments, the sonotrode 122 includes an ultrasonic converter which converts electrical energy to ultrasonic vibrations. The sonotrode 122 also includes an ultrasonic booster which amplifies the vibrations from the ultrasonic converter to a desired amplitude. The sonotrode 122 also includes an ultrasonic sonotrode or horn (e.g., two options with and without vacuum) which is the part of the sonotrode 122 that that physically oscillates to form the bond 160 between the webs 130, 140. In some embodiments, the anvil 124 is a vacuum anvil that is the part of the thermal bonding apparatus that receives the oscillation and holds the geometry shaped pattern of the bond 160.

After forming the splice bond 160 between the first and second portions 133, 143 of the expiring and new webs 130, 140, the system cuts excess material from the expiring web 130 (upstream of the splice location) and from the new web 140 (downstream of the splice location). FIG. 1C depicts a cut leading edge 150 from the expiring web 130 by the splice blade 120 upstream of the splice location and thus upstream of the splice bond 160, resulting in a cut trailing edge 131. FIG. 1C similarly depicts a cut tail 152 from the new web 140 by the outer layer blade 128 downstream of the splice location and thus downstream of the splice bond 160. In these embodiments, the controller 110 transmits signals to each of the splice blade 120 and the outer layer blade 128 to automatically cut the respective cut leading edge 150 and tail 152 after forming the splice bond 160. The cutting of the cut leading edge 150 and cut tail 152 advantageously removes undesired web material extended from the splice bond 160. In an example embodiment, the cut leading edge 150 has a length in the range of about 0.01 mm to about 20 mm. In another example embodiment, the cut tail 152 has a length in the range of about 0.01 mm to about 20 mm. In an example embodiment, the tail retraction and removal advantageously prevents issues in downstream conveying of the tail, and this is enabled by a special design of the vacuum bar 126 and ultrasonic sonotrode 122. In some embodiments, the use of the sensors and motors herein resolve to minimize the cut leading edge 150 and cut tail 152 length by eliminating the non-bonded tail to zero. In one example embodiment, the length of the cut tail 152 is minimized by using a vacuum sonotrode 122 or the vacuum bar 126 near the sonotrode 122.

As further shown in FIG. 1C, due to the continual splicing process, the splicer buffer system 144" of the expiring web 130 has reduced from the previous splicer buffer system 144' (FIG. 1B), 144 (FIG. 1A) during earlier steps in the process. This advantageously ensures continual operation of the manufacturing line 149 during the splicing process. The amount of the initial splicer buffer system 144 (FIG. 1A) is adjusted such that the product of a length of the splicer buffer system 144 and the average speed of the expiring web 130 is less than an estimated stoppage time of the expiring web 130 to perform the splicing process herein.

Although the previously discussed embodiments discuss that the first component and the second component of the thermal bonding apparatus are respectively the sonotrode 122 and the anvil 124, in other embodiments other types of thermal bonding apparatus components can be used to form the splice bond. In one example embodiment, the first and second components of the thermal bonding apparatus can feature a heat bar or a source of hot fluid.

The splice bond formed between the expiring and new webs will now be discussed. FIG. 1D is a block diagram that illustrates an example of the system 100 of FIG. 1A upon forming a splice bond 160 between the first portion 133 of the expiring web 130 and the second portion 143 of the new web 140. In some embodiments, the splice bond 160 is a thermal weld at the splice location between the first and second portion 133, 143 of the respective expiring and new webs 130, 140. In one embodiment, the splice bond 160 is not formed by the thermal welding apparatus traversing across the expiring web 130 and the new web 140. In another embodiment, the splice bond 160 is tapeless. In another example embodiment, the splice bond 160 is formed in an amount of time in a range from about 0.1 seconds to about 0.5 seconds. In some embodiments, the formation of the splice bond 160 may include an initial web stabilization step that takes an amount of time between about 0.05 seconds and about 0.1 seconds, followed by a welding step to form the splice bond 160 that takes an amount of time between about 0.1 seconds and about 0.5 seconds and a web cool-off step that takes an amount of time between about 0.05 seconds and about 0.2 seconds. In an example embodiment, a plunge splice is preferred over a traversing splice. Additionally, in an example embodiment, an ultrasonic thermal bonding apparatus (e.g. featuring the sonotrode 122) is preferred over thermal conduction heat (e.g. Hot Wire). The use of the ultrasonic welding apparatus advantageously leads to less accumulated material in the web buffer system and ensures a zero-speed splice can be achieved.

The system 100 advantageously conveys the new web 140 through the splice box 102, splice bonds the new web 140 to the expiring web 130 and conveys the new web 140 into the absorbent article manufacturing line 149 without stoppage of the absorbent article manufacturing line 149 during the splicing process. This is depicted in FIG. 1D where the splicer buffer system 144 of the expiring web 130 has reduced to zero without any stoppage in the absorbent article manufacturing line 149. It should be noted that the splicer buffer system 144 of the expiring web 130 need not be reduced to zero at the time that the splice bond 160 is formed but instead may be any non-zero value. Additionally, after the splice bond 160 is formed and the new web 140 is conveyed through the splice box 102 and to the absorbent article manufacturing line 149, the new web 140 is accumulated downstream of the splice box 102 to a similar extent as the splicer buffer system 144 of the expiring web 130 in FIG. 1A. This splicer buffer system of the new web 140 can then be used in the event of a future splicing between an expiring web 140 and a new web.

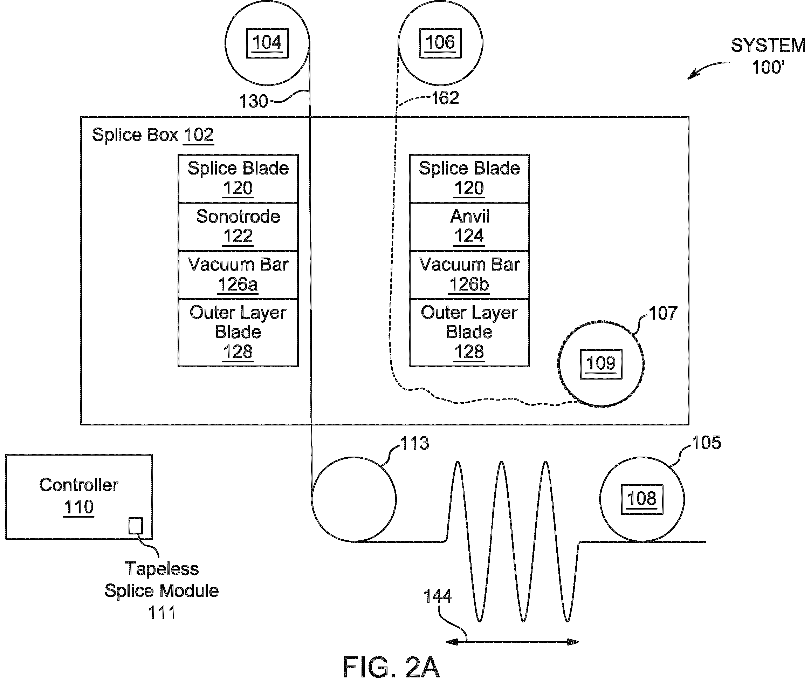

Although the embodiment of FIGS. 1A and 1B discuss one embodiment where unwanted outer layers of the new web 140 are manually removed, in other embodiments the unwanted outer layers of the new web 140 are automatically removed. FIGS. 2A through 2C discuss a system 100' that is similar to the system 100 of FIGS. 1A through 1D with the exception of the features discussed herein. The automated removal of the unwanted outer layers of the new web 140 increases the production hygiene standards, which include winding the outer layers of material off the new web 140 around a gripper, cutting the excess material using a cutter to separate the outer layers of the new web 140 from the splice tail. In an example embodiment, this could be achieved either by a Splicer mounted Cobot Arm or an AMR Cobot.

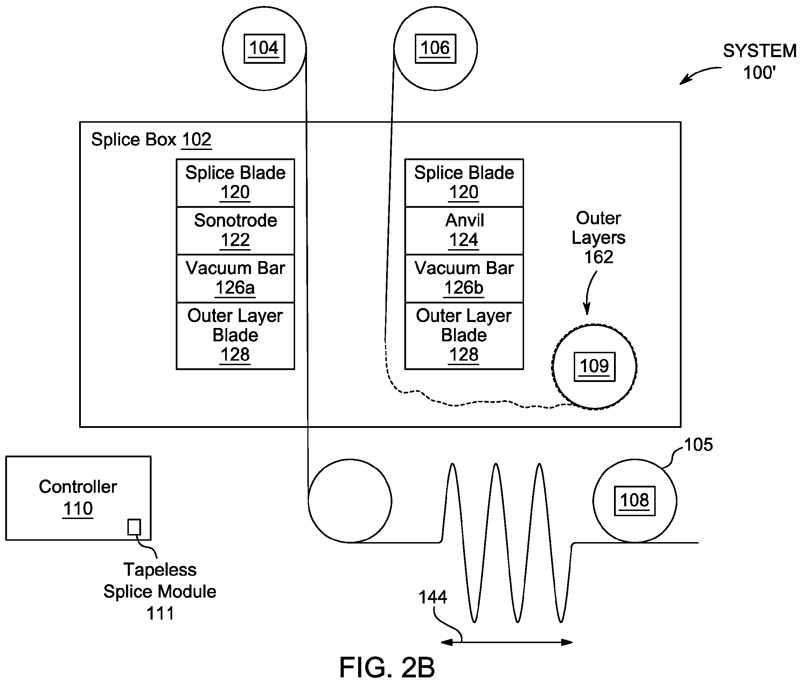

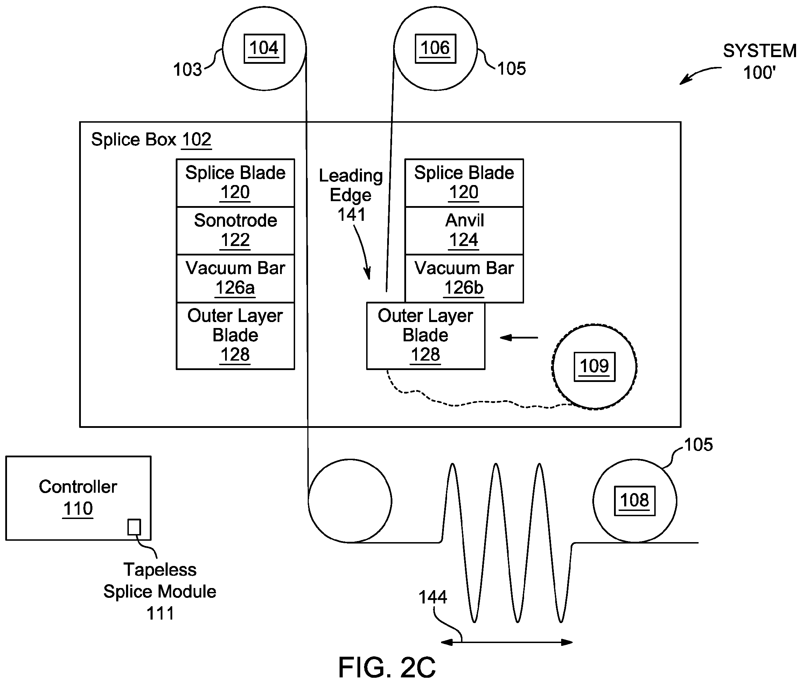

FIG. 2A is a block diagram that illustrates an example of a system 100' where outer layers 162 of the new web 140 are collected downstream of the splice location, according to various embodiments. As shown in FIG. 2A, the system 100' features a fourth mandrel 107 operated by a fourth motor 109. In some embodiments, the fourth mandrel 107 and fourth motor 109 are components of a robot that is moved into the splice box 102 and is used to collect the unwanted outer layers of the new web 140. The dotted line in FIG. 2A indicates unwanted outer layers 162 of the new web 140 which are to be removed prior to splicing the new web 140 to the expiring web 130. In this embodiment, as shown in FIG. 2A the motors 106, 109 are activated by the controller 110 in order to collect the outer layers 162 of the new web 140 on the fourth mandrel 107. The controller 110 activates the motors 106, 109 until the predetermined length of the outer layers 162 are collected on the fourth mandrel 107. FIG. 2B is a block diagram that illustrates the system 100' after the outer layers 162 of the new web 140 are downstream of the splice location and on the fourth mandrel 107. As shown in FIG. 2C, the outer layer blade 128 is then used to cut the outer layers 162 from the new web 140, and thus forming the leading edge 141 that is positioned proximate to and downstream of the anvil 124. The controller 110 then signals the motor 109 to rotate the third mandrel 103 in a clockwise direction to move the leading edge 141 in an upstream direction until the sensor (not shown) detects that it is within the threshold distance of the anvil 124. The new web 140 in the system 100' in FIG. 2C then resembles the new web 140 of the system 100 of FIG. 1B. The next steps of the system 100' in performing the splice bond 160 are similar to the other steps in the system 100 of FIGS. 1B through 1D that were previously discussed. These other steps would include the detection of the upcoming expiration of the expiring web 130 (FIG. 1A) and the control of the speed of the expiring web 130 until the first portion 133 is adjacent to the sonotrode 122.

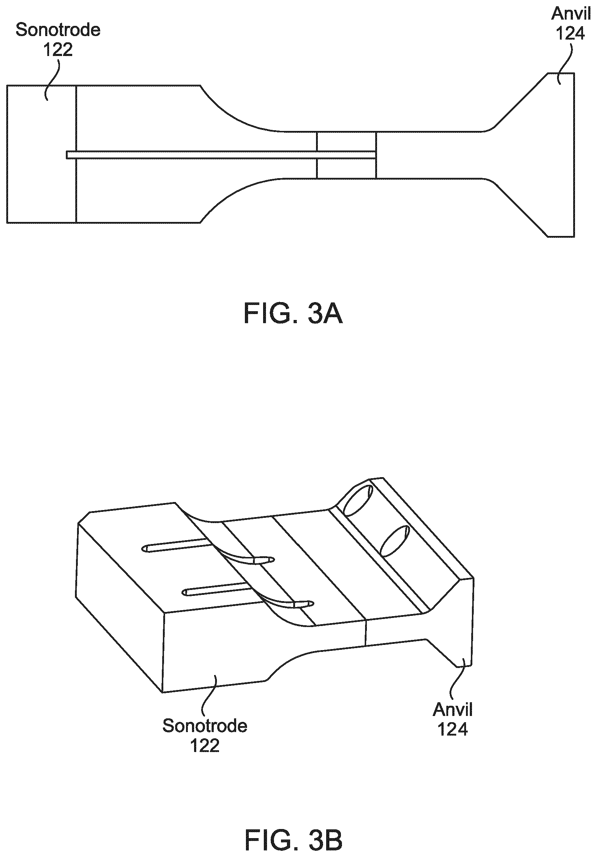

FIG. 3A is a side view that illustrates an example of the sonotrode 122 and anvil 124 of the system 100 of FIGS. 1C and 1D forming the splice bond 160, according to various embodiments. FIG. 3B is a perspective view that illustrates an example of the sonotrode 122 and anvil 124 of the system 100 of FIGS. 1C and 1D forming the splice bond 160, according to various embodiments.

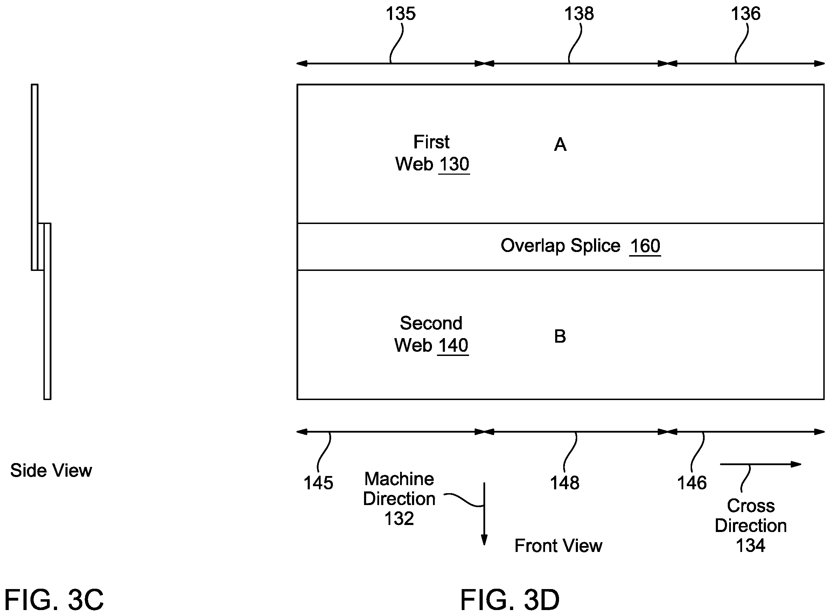

FIG. 3C is a side view that illustrates an example of the splice bond 160 formed between the expiring web 130 and the new web 140 in the system 100 of FIG. 1D, according to various embodiments. FIG. 3D is a front view that illustrates an example of the splice bond 160 formed between the expiring web 130 and the new web 140 in the system 100 of FIG. 1D, according to various embodiments. As shown in FIG. 3D, in some embodiments the expiring web 130 includes, in the CD direction 134, a first side region 135, a second side region 136 and a central region 138 between the first and second side regions 135, 136. The new web 140 similarly includes, in the CD direction 134, a first side region 145, a second side region 146 and a central region 148 between the first and second side regions 145, 146. In some embodiments, the thermal welding apparatus including the sonotrode 122 and the anvil 124 form the splice bond 160 across the first side region 135, second side region 136 portion 135 and central region 138 of the first portion 133 and across the first side region 145, second side region 146 and central region 148 of the second portion 143 at the same time. In an example embodiment, the splice bond 160 is formed using a full width plunge-style sonotrode 122 (e.g., distinguished over known CD traversing anvil splice box designs).

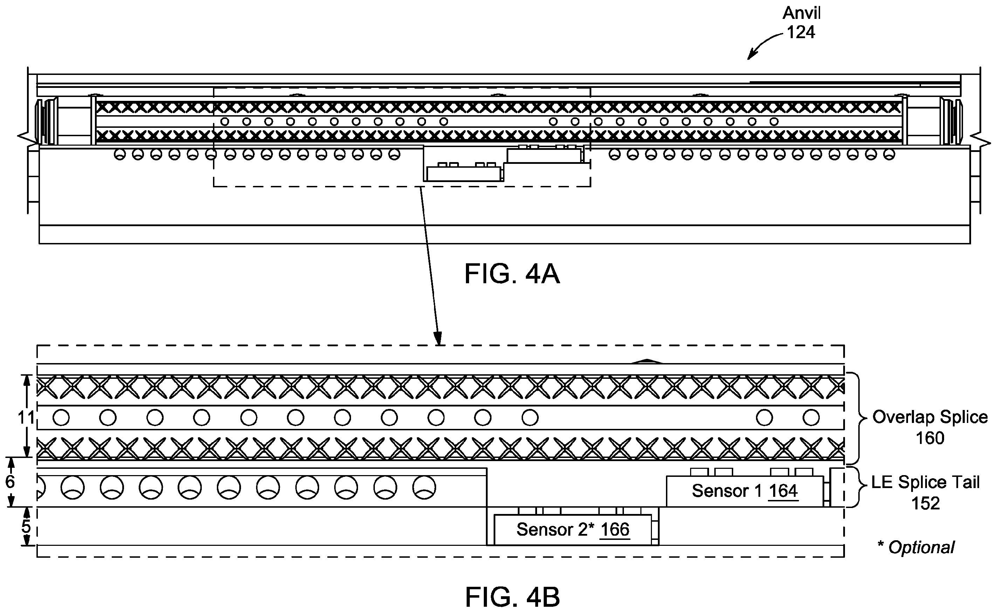

FIGS. 4A and 4B are front views that illustrates an example of the anvil 124 and adjacent sensors 164, 166 to detect a presence of the leading edge 141 of the new web 140 in the system 100 of FIG. 1B, according to various embodiments. In one embodiment, the anvil 124 of FIGS. 4A and 4B includes an integrated vacuum bar that performs the function of the vacuum bar 126b and features multiple sensors 164, 166 to assist with performing one or more steps by the system. In one example, the multiple sensors 164, 166 are web presence sensors that detect the presence of the web 140 (e.g. leading edge 141) proximate to the anvil 124. In another example, the multiple sensors 164, 166 are embedded optical sensors. In an embodiment, the sensors 164, 166 are communicatively coupled with the controller 110 and transmit signals to the controller 110 based on detecting a presence of the new web 140 (e.g. leading edge 141) at either sensor.

During a splice preparation process (e.g. prior to detection of the upcoming expiration of web 130), as the new web 140 is directed in the downstream direction, once the bottom web presence sensor 166 detects a presence of the new web 140, the bottom web presence sensor 166 may transmit a signal to the controller 110 which may cause the controller 110 to activate the vacuum (e.g. vacuum bar 126b) of the integrated vacuum bar. This would then cause the new web 140 to adhere to the anvil 124 of the integrated vacuum bar. Although this embodiment discuses an option where the controller 110 automatically activates the vacuum upon detection of the new web 140 by the bottom presence sensor 166, in other embodiments the user may manually activate the vacuum (e.g. pressing a reset button) upon the bottom presence sensor 166 initial detection of the new web 140.

Additionally, during the splice preparation process, after activation of the vacuum of the integrated vacuum bar to adhere the new web 140 to the anvil 124, the controller 110 may transmit a signal to the motor 106 of the mandrel 103 to move the new web 140 in the upstream direction. Thus, as the new web 140 is adhered to the anvil 124, the mandrel 103 rotates in the clockwise direction and causes the new web 140 to move in the upstream direction as it is adhered to the anvil 124. In one example, this upstream movement of the new web 140 may continue until the top web presence sensor 164 no longer detects the new web 140. This may indicate that the leading edge 141 of the new web 140 is adhered to the anvil 124 or that the leading edge 141 is positioned between the top web presence sensor 164 and the anvil 124. In this embodiment, upon receiving a signal from the top web presence sensor 164 that the new web 140 is no longer detected, the controller 110 may transmit a signal to the motor 106 of the mandrel 103 to slow down and/or stop the mandrel 103 movement in the upstream direction. Although this embodiment discuses an option where the controller 110 automatically causes the new web 140 to move in the upstream direction until the leading edge 141 is adhered to and/or in close proximity to the anvil 124, in other embodiments the user may manually cause the new web 140 to move in the upstream direction (e.g. pressing a reset button) until such time as the leading edge 141 is adhered to and/or in close proximity to the anvil 124, after which the user may manually cause the new web 140 to slow down and/or stop moving in the upstream direction (e.g. pressing a reset button).

Although the above embodiment discusses the use of two web presence sensors 164, 166, in other embodiments a single web presence sensor may be employed in the integrated vacuum bar. In this embodiment, the vacuum of the integrated vacuum bar may be activated based on the initial detection of the new web 140 by the web presences sensor (as the new web 140 moves in the downstream direction through the splice box 102). In this embodiment, after activation of the vacuum, the controller 110 may transmit a signal to the motor 106 of the mandrel 103 to move the new web 140 in the upstream direction (as the new web 140 is adhered to the anvil 124). Once the single web presence sensor no longer detects the presence of the new web 140, the controller 110 may signal the motor 106 to slow down and/or stop the upstream movement of the new web 140.

In an example embodiment, when the controller 110 receives a signal from the first sensor 166 indicating no presence of the new web 140 at the first sensor 166 but does receive a signal from the second sensor 164 indicating a presence of the new web 140 at the second sensor 164, this communicates to the controller 110 that the leading edge 141 is positioned within the threshold distance of the anvil 124. In this example embodiment, the controller 110 transmits a signal to the motor 106 of the third mandrel 103 to stop and/or slow down the moving the new web 140 in the upstream direction and thus fix the leading edge 141 at the threshold distance from the anvil 124. As previously discussed herein, upon forming the splice bond 160, the controller 110 may transmit a signal to deactivate the vacuum of the integrated vacuum bar and/or a signal to the outer layer blade 128 to cut the tail 152 of the new web 140 which is based on the extent to which the new web 140 extends from the anvil 124 over the second sensor 164.

In some embodiments, zero-tail can be achieved by requiring that both sensors 164, 166 do not detect the new web 140 which corresponds to the new web 140 having moved such that the leading edge 141 is within the anvil 124 (e.g. at the boundary between the anvil 124 and the sensor 164). In other embodiments, the sensors 164, 166 are provided along the anvil 124 and thus zero tail can be achieved (e.g. when the new web 140 is cut within the anvil 124 and thus no tail is formed beyond the splice bond 160). In yet another embodiment, the sensors 164, 166 of FIG. 4B can be utilized to achieve zero-tail, where after the sensor 164 detects the new web 140 but the sensor 166 does not detect the new web 140, the controller 110 transmits a signal to the motor 106 of the mandrel 103 to move the new web 140 in the upstream direction by a fixed distance (e.g. the thickness of the sensor 164 in the MD direction 132 and/or the detection range of the sensor 164 in the MD direction 132). Then the leading edge 141 of the new web 140 would be moved to a boundary between the anvil 124 and the sensor 164 and thus the splice bond 160 would be formed with zero-tail downstream of the splice location. In an example embodiment, the sensors 164, 166 are offset in the CD direction 134 which advantageously enhances the accessibility of each sensor (e.g. in the event of repair and/or replacement).

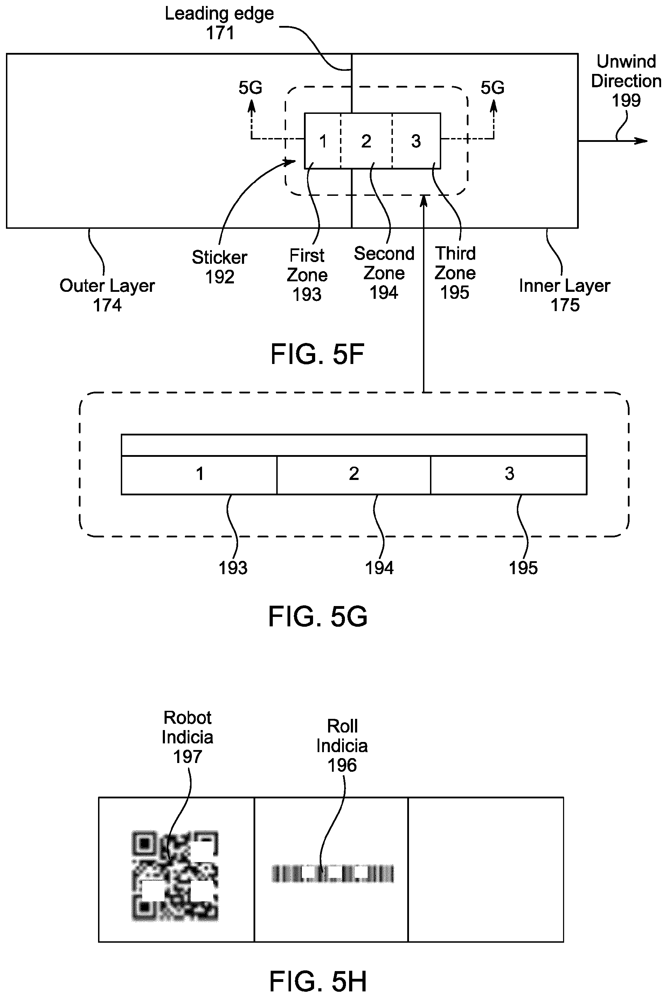

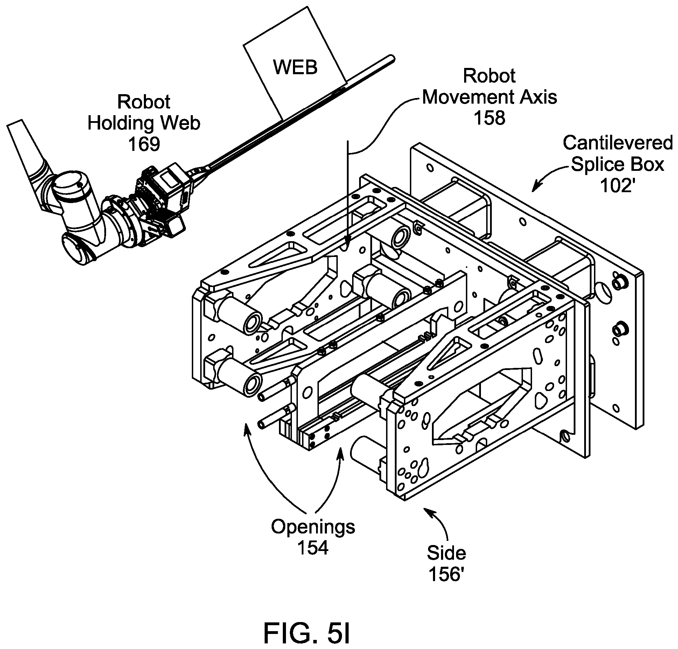

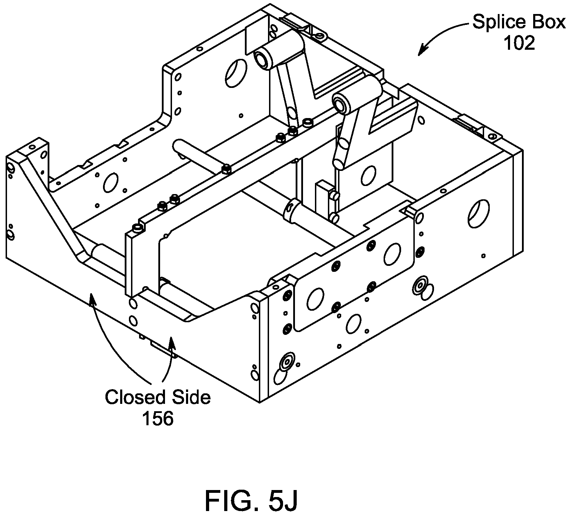

In an embodiment, the splice box 102 is automated such that the expiring web 130 and/or the new web 140 are guided into the splice box by one or more robots. In one example embodiment, such robots include the aspect of the automated removal of the unwanted outer layers 162 of the new web 140 (FIGS. 2A through 2C). Additionally, as shown in FIG. 5I, in another example embodiment, a cantilevered/side-open splice box 102' design is provided. The cantilevered splice box 102' has a side 156' facing an operator that defines one or more openings 154. These openings 154 align with a movement axis 158 of a robot 169 (e.g. holding the new web 140) such that the robot can automatically move along the movement axis 158 into the splice box 102'. This embodiment of the cantilevered splice box 102' advantageously allows a robotic thread up arm to penetrate the splice box 102' from one side to enable web thread up. In one embodiment, the robot 169 automatically picks up the leading edge of the new web 140 and automatically moves along the movement axis 158 through the opening 154 of the cantilevered splice box 102'. In some embodiments, once inside the splice box 102' the robot 169 automatically attaches the leading edge of the new web 140 to the mandrel 107 with the motor 109 such that the controller 110 can then transmit a signal to the motor 109 to collect the outer layers 162 of the new web 140 on the mandrel 107. The outer layer blade 128 then cuts the unwanted outer layers 162 from the new web 140. In other embodiments, the robot 169 includes the mandrel 107 and the motor 109 and moves the mandrel 107 (with the attached leading edge of the new roll 140) and the motor 109 into the splice box 102' after which the robot 169 activates the motor 109 to collect the unwanted layers 162 of the new roll 140 on the mandrel 107. The outer layer blade 128 of the splice box 102' is then activated by the controller 110 to cut the unwanted outer layers 162 from the new roll 140. The robot 169 then moves out of the splice box 102' through one of the openings 154 along the movement axis 158. The cantilevered splice box 102' of FIG. 5I is distinct from a non-cantilevered splice box 102 (FIG. 5J) where the side 156 of the splice box 102 does not feature an opening aligned with the robot movement axis 158 and thus automated motion of the robot 169 into the non-cantilevered splice box 102 is not possible due to a lack of access of the robot 169 along the movement axis 158 into the splice box 102. Yet further, in another example embodiment, such robots can be used for an automated mechanism for picking up the leading edge 141 of the new web 140. These embodiments of a robotic component used to automatically convey the leading edge 141 of the new web 140 into the splice box 102 will now be discussed.

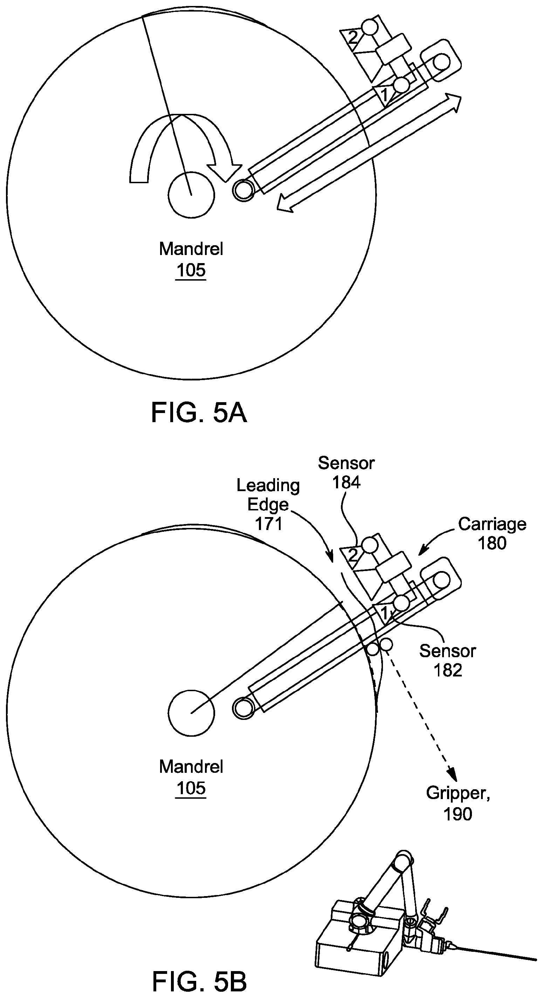

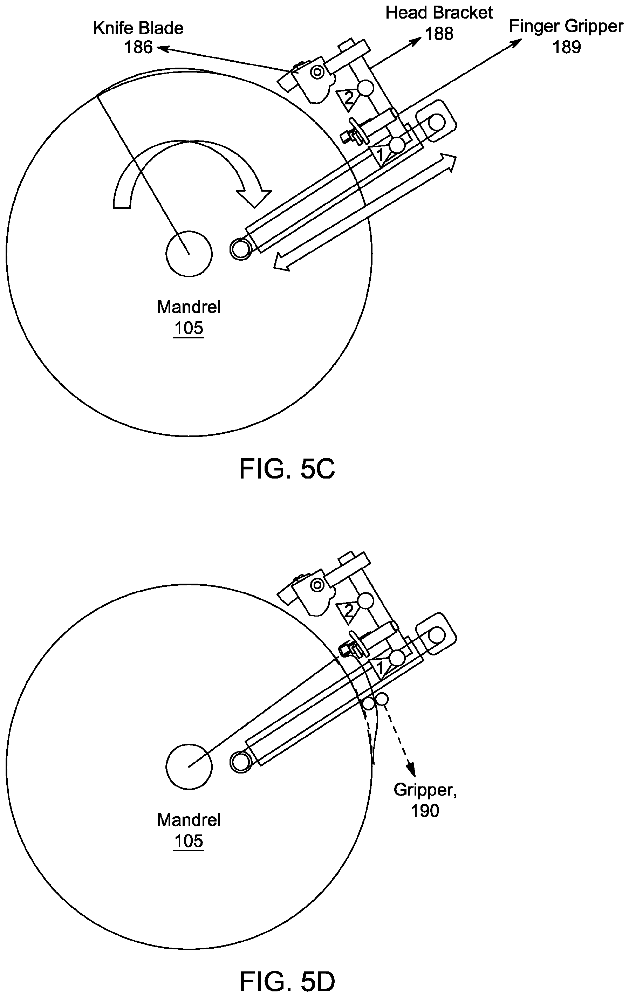

Some embodiments of a device are designed to enable automated pick up the start of the roll (e.g. leading edge of the new web 140). This is a challenging task without tape as the system needs to distinguish between materials of the same color and texture. Two design options are illustrated one with the use of vacuum (FIGS. 5A and 5B), and a second with the use of air blade and mechanical finger gripper (FIGS. 5C and 5D).

FIGS. 5A and 5B are side views that illustrates an example of the mandrel 103 of the new web 140 and a vacuum device to automatically pick up the leading edge 171 of the new second web 140. In some embodiments, the leading edge 171 of FIG. 5B is different from the leading edge 141 of FIG. 2C, since the leading edge 171 of the new web 140 includes the outer layers 162 which are to be removed from the new roll 140. However, in other embodiments, the leading edge 171 is the same as the leading edge 141 of FIG. 1B, since in these embodiments the outer layers 162 were already manually removed from the new roll 140 prior to the automated pickup of the leading edge 171. In an example embodiment, the vacuum device is a pecking vacuum device. In this embodiment, the vacuum device includes a carriage 180 with a pair of spaced apart sensors 182, 184. The sensors 182, 184 detect a presence of the leading edge 171 of the new web 140 based on the first sensor 182 detecting the second web 140 at a certain radial position but the second sensor 184 not detecting the new web 104 at the same radial position. Based on the detection of the leading edge 171 via the sensors 182, 184, the vacuum device is configured to automatically pick up the leading edge 171 and convey the leading edge 171 to the gripper 190 which is then used to feed the leading edge 171 to the splice box 102'.

FIGS. 5C and 5D are side views that illustrates an example of the mandrel 103 of the new web 140 and an air blade 186 and mechanical finger 189 to automatically pick up the leading edge 171 of the new web 140. In an example embodiment, this involves mechanical friction and an airfoil effect for tail pickup. This embodiment also features the sensors 182, 184 that automatically detect the presence of the leading edge 171 of the new web 140. Based on detection of the leading edge 171, the knife blade 186 and finger gripper 189 operate to mechanically grip the leading edge 171 which is then automatically fed to the splice box 102'.

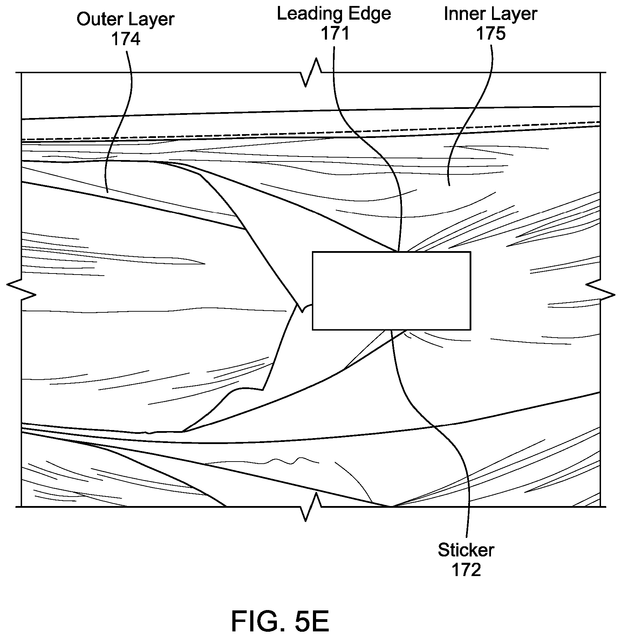

In addition to the previously discussed embodiments, another option for automated pickup of the leading edge 171 of the new web 140 from the roll involves leveraging a modified outer layer sticker (e.g., featuring a non-sticky zone) to identify and pick up the leading edge 171 of the new web 140. FIG. 5E depicts a conventional sticker 172 that is used to attach the leading edge 171 to the new web 140. As shown in FIG. 5E, the conventional sticker 172 adheres to the new web 140 on both sides of the leading edge 171 so to secure the leading edge 171 to the remaining layers 175 of the new web 140. However, the inventors of the present invention recognized that this conventional sticker 172 introduces a drawback in terms of automated pick up of the leading edge 171 with a robot. As shown in FIG. 5E, the conventional sticker 172 is fully adhered to both an outer layer 174 and a first inner layer 175 of the new web 140 across the leading edge 171. Thus, the inventors noticed that the leading edge 171 is difficult to pick up with an automated robot device (e.g. vacuum device) since the vacuum force of the automated device would need to overcome the relatively large force that the sticker 172 adheres the leading edge 171 to the new web 140.

To overcome the above noted drawback with conventional stickers 172, the inventors developed the multi-zone sticker 192 depicted in FIGS. 5F through 5H. Unlike the conventional sticker 172 that is fully adhered to both the outer layer 174 and the first inner layer 175 across the leading edge 171 of the new web 140, the multi-zone sticker 192 features multiple zones which adhere to the new web 140 with different levels of force. For purposes of this description, the "level of force" that a sticker adheres to a surface means an amount of force that would be required to separate the sticker from the surface. As shown in FIG. 5F, in one embodiment the multi-zone sticker 192 includes a first zone 193 that adheres to the outer layer 174 with a first level of force, a second zone 194 which adheres across the leading edge 171 (to both the outer layer 174 and the first inner layer 175 across the leading edge 171) with a second level of force that is less than the first level of force and a third zone 195 that is not adhered to the second web 140. In an example embodiment, the first zone 193 is a strong adhesive zone (e.g., adhered to the new web 140 with the first level of force, as defined herein), the second zone 194 is a mild adhesive zone (e.g. adhered to the new web 140 with the second level of force that is less than the first level of force) and the third zone 195 is a no glue and pickup zone (e.g. not adhered to the new web 140).

The gripper shown in Fig. 5I may grip the web in Fig. 5F in the third zone 195 (no adhesive and pick up zone) of the multizone sticker 192 and across the web in the cross-direction. The gripper will then begin to rotate to wind the web, but may do so in a way in which the exposed adhesive in the second zone 194 only contacts the web being wound onto itself and not contract portions of the gripper as the adhesive would stick to the gripper. As such, the adhesive in the second zone 194 of the sticker 192 will be on the side of the web most distal from the gripper while the side of the web opposite the side of the web that the sticker is on will be most proximal to the gripper during winding.

The multi-zone sticker 192 facilitates automated pick up of the leading edge 171 of the new web 140 with a robot device (e.g. vacuum device). In some embodiments, the robot 169 (e.g. vacuum device) adheres to the third zone 195 of the multi-zone sticker 192 and moves in the unwind direction 199 (e.g. corresponding to the robot movement axis 158 into the splice box 102'). Since the force of movement of the robot 169 in the unwind direction 199 is greater than the second level of force that the second zone 194 adheres across the leading edge 171, movement of the robot 169 (and attached third zone 195) causes the second zone 194 of the sticker 192 to detach from across the leading edge 171. Further movement of the robot 169 (and attached third zone 195) in the unwind direction 199 continues. The force of movement of the robot 169 in the unwind direction 199 does not exceed the first level of force that the first zone 193 is attached to the outer layer 174 of the new web 140. Thus, the first zone 193 of the sticker 192 does not detach from the outer layer 174 and thus the continued movement of the robot 169 in the unwind direction 199 causes the first zone 193 (and attached leading edge 171 to the first zone 193) to move with the robot in the unwind direction 199. In some embodiments, after the robot 169 moves the multi-zone sticker 192 and attached leading edge 171 through the opening 154 of the splice box 102', the robot 169 attaches the leading edge 171 to the mandrel 107 so that the controller 110 can activate the motor 109 and collect the unwanted outer layers 162 of the new web 140 on the mandrel 107. The outer layer blade 128 is then used to cut the unwanted outer layers 162 from the new web 140.

FIG. 5H depicts that in one embodiment, indicia can be provided in one or more zones of the multi-zone sticker 192. In one example embodiment, a robot indicia 197 is provided in one the zones (e.g. first zone 193). In this example embodiment, the robot 169 includes a scanning device that can scan the robot indicia 197 after which the robot 169 can use the scanned indicia 197 to verify that the robot 169 is assigned to pick up the leading edge 171 of the new web 140 with the scanned indicia 197. In a similar example embodiment, a roll indicia 196 is provided in one or more zones (e.g. second zone 194). In this example embodiment, the robot 169 includes the scanning device that can scan the roll indicia 196 after which the robot 169 can use the scanned indicia 196 to verify that the new web 140 having the scanned indicia 196 is in a database of new web rolls assigned to that particular robot 169 for automated pick up of the leading edge 171.

Although FIGS. 5F through 5H depict the multi-zone sticker 192 with three different zones, in other embodiments less or more than three zones are provided in the multi-zone sticker 192. For example, two zones can be provided in the multi-zone sticker 192 (e.g. a first zone that sticks to the new web 140 and a second zone that is not attached to the new web 140).

In an embodiment, the system 100, 100' involves an ultrasonic splice box 102 design, that is aiming to connect the expiring web 130 with the new web 140 via an ultrasonic overlap splice (plunge splice). This advantageously eliminates the need to have an adhesive tape. Additionally, the disclosed embodiments herein enable an automated thread up solution for existing robots and/or other automated thread up systems (e.g. the automated outer layer removal from the new web 140, as shown in FIGS. 2A though 2C).

The system 100, 100' disclosed herein has different operating modes. In a first operating mode, the system is operated with a vacuum bar and material length (e.g. rotation controlled). In a second operating mode, the system is operated with a vacuum bar and sensor. In a third operating mode, the system is operated with a vacuum bar and a vacuum sonotrode/anvil. In a fourth operating mode, the system is operated with a vacuum bar, a vacuum sonotrode/anvil and an embedded sensor.

### Method for Forming a Tapeless Splice Bond

FIG. 6 is a flowchart that illustrates an example of a method 200 for forming a tapeless splice bond 160 between material of the expiring web 130 and material of the new web 140, according to various embodiments. Although the flow diagram of FIG. 6 is depicted as integral steps in a particular order for purposes of illustration, in other embodiments one or more steps, or portions thereof, are performed in a different order, or overlapping in time, in series or in parallel, or are deleted, or one or more other steps are added, or the method is changed in some combination of ways.

In step 201, the expiring web 130 is conveyed through the splice box 102 and into the absorbent manufacturing line 149. As previously discussed, in step 201 the controller 110 transmits signals to the motor 104 of the first mandrel 101 such that the expiring web 130 is continuously fed through the splice box 120 and to the manufacturing line 149.

In step 202, the leading edge 141 of the new web 140 is conveyed through the splice box 102. In one embodiment, as shown in FIG. 1A, the leading edge 141 is conveyed past the splice location after which the controller 110 signals the vacuum bar 126b to adhere the new web 140 to the second component (e.g. anvil 124) of the thermal bonding apparatus. The controller 110 may then output a signal to the motor 106 of the mandrel 103 to move the new web 140 (now adhered to the anvil 124) in the upstream direction (opposite to the MD direction 132) until the leading edge 141 is within a threshold distance of the second component (e.g. anvil 124) of the thermal bonding apparatus and/or the second portion 143 of the new web 140 is adjacent to the second component of the thermal bonding apparatus. The controller 110 then signals the motor 106 of the mandrel 103 to stop and/or slow down rotation of the mandrel 103 and thus fix the second portion 143 of the new web 140 adjacent to the second component (e.g. anvil 124) of the thermal bonding apparatus.

In step 204, upon sensing the upcoming expiration of the first web 130, the speed of the expiring web 130 is adjusted. In an embodiment, in step 204 the controller 110 receives a signal from the sensor (not shown) that detects the upcoming expiration of the web 130. In one example embodiment, the sensor is on the mandrel 101 of the expiring web 130 and detects when a number of remaining layers (e.g. radial thickness) of the expiring web 130 falls below a threshold value. In another example embodiment, the sensor is an imaging device (e.g. camera) that detects that the number of remaining layers of the expiring web 130 on the mandrel 101 has fallen below the threshold value. As previously discussed, in step 204 upon detecting the upcoming expiration of the web 130, the controller 110 transmits signals to the motor 104 to slow down the rotation speed of the mandrel 101 and the expiring web 130 thereon. When the first portion 133 of the expiring web 130 is positioned adjacent to the first component (e.g. sonotrode 122) of the thermal welding apparatus, the controller 110 transmits a signal to stop and/or slow down the motor 104 and thus to fix the first portion 133 at the first component (e.g. sonotrode 122) of the thermal welding apparatus. In some embodiments, in step 204 the motor 108 of the mandrel 105 does not stop and thus the manufacturing line 149 is continuously fed from the splicer buffer system 144 of the expiring web 130. In still other embodiments, in step 204 upon detecting the upcoming expiration of the web 130, the controller 110 transmits signals to the motor 104 to speed up the rotation speed of the mandrel 101 and the expiring web 130 thereon, to increase the accumulation of the splicer buffer system 144 downstream of the splice box 102.

In step 206, the thermal weld or splice bond 160 is formed between the expiring web 130 and the new web 140. In one embodiment, in step 206 the splice bond 160 is formed between the first portion 133 of the expiring web 130 and the second portion 143 of the new web 140. In this embodiment, in step 206 the controller 110 signals a motor (not shown) of the thermal bonding apparatus to move the first and second components (e.g. sonotrode 122 and anvil 124), as well as the vacuum bars 126a, 126b, together in a direction orthogonal to the MD direction 132. The controller 110 also signals the thermal bonding apparatus to activate the sonotrode 122 and anvil 124 to form the splice bond 160. As previously discussed, the first portion 133 of the expiring web 130 and the second portion 143 of the new web 140 have an overlapping configuration (in the MD direction 132) when step 206 is performed. In an embodiment, in step 206 the sonotrode 122 vibrates against the anvil 124 to form the splice bond 160. As previously discussed, in step 202 the vacuum bar 126b was activated to adhere the second portion 143 of the new web 140 to the anvil 124, prior step 206 of the controller 110 transmitting the signals to move the sonotrode 122 and anvil 124 together to form the splice bond 160.

In step 208, either prior to or after step 206, the expiring web 130 is cut with the splice blade 120 upstream of the splice location to form the cut leading edge 150 upstream of the splice location or splice bond 160. Additionally, in step 208 the new web 140 is cut with the outer layer blade 128 downstream of the splice location to cut the tail 152 off the new web 140 downstream of the splice location or splice bond 160. Step 208 advantageously removes excess web material (e.g., the cut leading edge 150 and cut tail 152) from the splice bond 160.

In step 210, after the splice bond 160 is formed the controller 110 signals the motor 106 of the mandrel 103 to convey the new web 140 (now attached to the expiring web 130 via. the splice bond 160) through the splice box 102 and to the absorbent article manufacturing line 149. Motor 108 of the mandrel 105 is not activated in step 210 as it remains activated during the method 200 steps.

In an embodiment, the steps of the method 200 are performed without stoppage of the absorbent article manufacturing line 149 and thus either the expiring web 130 or new web 140 is continuously fed to the absorbent article manufacturing line 149 during the method 200. In an example embodiment, this is achieved in part due to the splicer buffer system 144 of the expiring web 130 that is provided prior to performing the method 200. Specifically, the steps of the method 200 that involve slowing down the expiring web 130, fixing the first and second portions 133, 143 at the thermal bonding apparatus, forming the splice bond 160 and then accelerating the new web 140 through the splice box 102. These steps are collectively performed over a time period that is less than the time period that the splicer buffer system 144 of the expiring web 130 can continuously feed the absorbent article manufacturing line 149.

### Hardware

FIG. 7 is a block diagram that illustrates a computer system 300 upon which an embodiment of the invention may be implemented. Computer system 300 includes a communication mechanism such as a bus 310 for passing information between other internal and external components of the computer system 300. Information is represented as physical signals of a measurable phenomenon, typically electric voltages, but including, in other embodiments, such phenomena as magnetic, electromagnetic, pressure, chemical, molecular atomic and quantum interactions. For example, north and south magnetic fields, or a zero and non-zero electric voltage, represent two states (0, 1) of a binary digit (bit). Other phenomena can represent digits of a higher base. A superposition of multiple simultaneous quantum states before measurement represents a quantum bit (qubit). A sequence of one or more digits constitutes digital data that is used to represent a number or code for a character. In some embodiments, information called analog data is represented by a near continuum of measurable values within a particular range. Computer system 300, or a portion thereof, constitutes a means for performing one or more steps of one or more methods described herein.

A sequence of binary digits constitutes digital data that is used to represent a number or code for a character. A bus 310 includes many parallel conductors of information so that information is transferred quickly among devices coupled to the bus 310. One or more processors 302 for processing information are coupled with the bus 310. A processor 302 performs a set of operations on information. The set of operations include bringing information in from the bus 310 and placing information on the bus 310. The set of operations also typically include comparing two or more units of information, shifting positions of units of information, and combining two or more units of information, such as by addition or multiplication. A sequence of operations to be executed by the processor 302 constitutes computer instructions.

Computer system 300 also includes a memory 304 coupled to bus 310. The memory 304, such as a random access memory (RAM) or other dynamic storage device, stores information including computer instructions. Dynamic memory allows information stored therein to be changed by the computer system 300. RAM allows a unit of information stored at a location called a memory address to be stored and retrieved independently of information at neighboring addresses. The memory 304 is also used by the processor 302 to store temporary values during execution of computer instructions. The computer system 300 also includes a read only memory (ROM) 306 or other static storage device coupled to the bus 310 for storing static information, including instructions, that is not changed by the computer system 300. Also coupled to bus 310 is a non-volatile (persistent) storage device 308, such as a magnetic disk or optical disk, for storing information, including instructions, that persists even when the computer system 300 is turned off or otherwise loses power.

Information, including instructions, is provided to the bus 310 for use by the processor from an external input device 312, such as a keyboard containing alphanumeric keys operated by a human user, or a sensor. A sensor detects conditions in its vicinity and transforms those detections into signals compatible with the signals used to represent information in computer system 300. Other external devices coupled to bus 310, used primarily for interacting with humans, include a display device 314, such as a cathode ray tube (CRT) or a liquid crystal display (LCD), for presenting images, and a pointing device 316, such as a mouse or a trackball or cursor direction keys, for controlling a position of a small cursor image presented on the display 314 and issuing commands associated with graphical elements presented on the display 314.

In the illustrated embodiment, special purpose hardware, such as an application specific integrated circuit (IC) 320, is coupled to bus 310. The special purpose hardware is configured to perform operations not performed by processor 302 quickly enough for special purposes. Examples of application specific ICs include graphics accelerator cards for generating images for display 314, cryptographic boards for encrypting and decrypting messages sent over a network, speech recognition, and interfaces to special external devices, such as robotic arms and medical scanning equipment that repeatedly perform some complex sequence of operations that are more efficiently implemented in hardware.

Computer system 300 also includes one or more instances of a communications interface 370 coupled to bus 310. Communication interface 370 provides a two-way communication coupling to a variety of external devices that operate with their own processors, such as printers, scanners and external disks. In general, the coupling is with a network link 378 that is connected to a local network 380 to which a variety of external devices with their own processors are connected. For example, communication interface 370 may be a parallel port or a serial port or a universal serial bus (USB) port on a personal computer. In some embodiments, communications interface 370 is an integrated services digital network (ISDN) card or a digital subscriber line (DSL) card or a telephone modem that provides an information communication connection to a corresponding type of telephone line. In some embodiments, a communication interface 370 is a cable modem that converts signals on bus 310 into signals for a communication connection over a coaxial cable or into optical signals for a communication connection over a fiber optic cable. As another example, communications interface 370 may be a local area network (LAN) card to provide a data communication connection to a compatible LAN, such as Ethernet. Wireless links may also be implemented. Carrier waves, such as acoustic waves and electromagnetic waves, including radio, optical and infrared waves travel through space without wires or cables. Signals include man-made variations in amplitude, frequency, phase, polarization or other physical properties of carrier waves. For wireless links, the communications interface 370 sends and receives electrical, acoustic or electromagnetic signals, including infrared and optical signals, that carry information streams, such as digital data.

The term computer-readable medium is used herein to refer to any medium that participates in providing information to processor 302, including instructions for execution. Such a medium may take many forms, including, but not limited to, non-volatile media, volatile media and transmission media. Non-volatile media include, for example, optical or magnetic disks, such as storage device 308. Volatile media include, for example, dynamic memory 304. Transmission media include, for example, coaxial cables, copper wire, fiber optic cables, and waves that travel through space without wires or cables, such as acoustic waves and electromagnetic waves, including radio, optical and infrared waves. The term computer-readable storage medium is used herein to refer to any medium that participates in providing information to processor 302, except for transmission media.

Common forms of computer-readable media include, for example, a floppy disk, a flexible disk, a hard disk, a magnetic tape, or any other magnetic medium, a compact disk ROM (CD-ROM), a digital video disk (DVD) or any other optical medium, punch cards, paper tape, or any other physical medium with patterns of holes, a RAM, a programmable ROM (PROM), an erasable PROM (EPROM), a FLASH-EPROM, or any other memory chip or cartridge, a carrier wave, or any other medium from which a computer can read. The term non-transitory computer-readable storage medium is used herein to refer to any medium that participates in providing information to processor 302, except for carrier waves and other signals.

Logic encoded in one or more tangible media includes one or both of processor instructions on a computer-readable storage media and special purpose hardware, such as ASIC *320.

Network link 378 typically provides information communication through one or more networks to other devices that use or process the information. For example, network link 378 may provide a connection through local network 380 to a host computer 382 or to equipment 384 operated by an Internet Service Provider (ISP). ISP equipment 384 in turn provides data communication services through the public, world-wide packet-switching communication network of networks now commonly referred to as the Internet 390. A computer called a server 392 connected to the Internet provides a service in response to information received over the Internet. For example, server 392 provides information representing video data for presentation at display 314.

The invention is related to the use of computer system 300 for implementing the techniques described herein. According to one embodiment of the invention, those techniques are performed by computer system 300 in response to processor 302 executing one or more sequences of one or more instructions contained in memory 304. Such instructions, also called software and program code, may be read into memory 304 from another computer-readable medium such as storage device 308. Execution of the sequences of instructions contained in memory 304 causes processor 302 to perform the method steps described herein. In alternative embodiments, hardware, such as application specific integrated circuit 320, may be used in place of or in combination with software to implement the invention. Thus, embodiments of the invention are not limited to any specific combination of hardware and software.

The signals transmitted over network link 378 and other networks through communications interface 370, carry information to and from computer system 300. Computer system 300 can send and receive information, including program code, through the networks 380, 390 among others, through network link 378 and communications interface 370. In an example using the Internet 390, a server 392 transmits program code for a particular application, requested by a message sent from computer 300, through Internet 390, ISP equipment 384, local network 380 and communications interface 370. The received code may be executed by processor 302 as it is received, or may be stored in storage device 308 or other non-volatile storage for later execution, or both. In this manner, computer system 300 may obtain application program code in the form of a signal on a carrier wave.

Various forms of computer readable media may be involved in carrying one or more sequence of instructions or data or both to processor 302 for execution. For example, instructions and data may initially be carried on a magnetic disk of a remote computer such as host 382. The remote computer loads the instructions and data into its dynamic memory and sends the instructions and data over a telephone line using a modem. A modem local to the computer system 300 receives the instructions and data on a telephone line and uses an infra-red transmitter to convert the instructions and data to a signal on an infra-red a carrier wave serving as the network link 378. An infrared detector serving as communications interface 370 receives the instructions and data carried in the infrared signal and places information representing the instructions and data onto bus 310. Bus 310 carries the information to memory 304 from which processor 302 retrieves and executes the instructions using some of the data sent with the instructions. The instructions and data received in memory 304 may optionally be stored on storage device 308, either before or after execution by the processor 302.

FIG. 8 illustrates a chip set 400 upon which an embodiment of the invention may be implemented. Chip set 400 is programmed to perform one or more steps of a method described herein and includes, for instance, the processor and memory components described with respect to FIG. *3 incorporated in one or more physical packages (e.g., chips). By way of example, a physical package includes an arrangement of one or more materials, components, and/or wires on a structural assembly (e.g., a baseboard) to provide one or more characteristics such as physical strength, conservation of size, and/or limitation of electrical interaction. It is contemplated that in certain embodiments the chip set can be implemented in a single chip. Chip set 400, or a portion thereof, constitutes a means for performing one or more steps of a method described herein.

In one embodiment, the chip set 400 includes a communication mechanism such as a bus 401 for passing information among the components of the chip set 400. A processor 403 has connectivity to the bus 401 to execute instructions and process information stored in, for example, a memory 405. The processor 403 may include one or more processing cores with each core configured to perform independently. A multi-core processor enables multiprocessing within a single physical package. Examples of a multi-core processor include two, four, eight, or greater numbers of processing cores. Alternatively or in addition, the processor 403 may include one or more microprocessors configured in tandem via the bus 401 to enable independent execution of instructions, pipelining, and multithreading. The processor 403 may also be accompanied with one or more specialized components to perform certain processing functions and tasks such as one or more digital signal processors (DSP) 407, or one or more application-specific integrated circuits (ASIC) 409. A DSP 407 typically is configured to process real-world signals (e.g., sound) in real time independently of the processor 403. Similarly, an ASIC 409 can be configured to performed specialized functions not easily performed by a general purposed processor. Other specialized components to aid in performing the inventive functions described herein include one or more field programmable gate arrays (FPGA) (not shown), one or more controllers (not shown), or one or more other special-purpose computer chips.

The processor 403 and accompanying components have connectivity to the memory 405 via the bus 401. The memory 405 includes both dynamic memory (e.g., RAM, magnetic disk, writable optical disk, etc.) and static memory (e.g., ROM, CD-ROM, etc.) for storing executable instructions that when executed perform one or more steps of a method described herein. The memory 405 also stores the data associated with or generated by the execution of one or more steps of the methods described herein.

FIG. 9 is a diagram of exemplary components of a mobile terminal 500 (e.g., cell phone handset) for communications, which is capable of operating in the system of FIG. 2C, according to one embodiment. In some embodiments, mobile terminal 501, or a portion thereof, constitutes a means for performing one or more steps described herein. Generally, a radio receiver is often defined in terms of front-end and back-end characteristics. The front-end of the receiver encompasses all of the Radio Frequency (RF) circuitry whereas the back-end encompasses all of the base-band processing circuitry. As used in this application, the term "circuitry" refers to both: (1) hardware-only implementations (such as implementations in only analog and/or digital circuitry), and (2) to combinations of circuitry and software (and/or firmware) (such as, if applicable to the particular context, to a combination of processor(s), including digital signal processor(s), software, and memory(ies) that work together to cause an apparatus, such as a mobile phone or server, to perform various functions). This definition of "circuitry" applies to all uses of this term in this application, including in any claims. As a further example, as used in this application and if applicable to the particular context, the term "circuitry" would also cover an implementation of merely a processor (or multiple processors) and its (or their) accompanying software/or firmware. The term "circuitry" would also cover if applicable to the particular context, for example, a baseband integrated circuit or applications processor integrated circuit in a mobile phone or a similar integrated circuit in a cellular network device or other network devices.

Pertinent internal components of the telephone include a Main Control Unit (MCU) 503, a Digital Signal Processor (DSP) 505, and a receiver/transmitter unit including a microphone gain control unit and a speaker gain control unit. A main display unit 507 provides a display to the user in support of various applications and mobile terminal functions that perform or support the steps as described herein. The display 507 includes display circuitry configured to display at least a portion of a user interface of the mobile terminal (e.g., mobile telephone). Additionally, the display 507 and display circuitry are configured to facilitate user control of at least some functions of the mobile terminal. An audio function circuitry 509 includes a microphone 511 and microphone amplifier that amplifies the speech signal output from the microphone 511. The amplified speech signal output from the microphone 511 is fed to a coder/decoder (CODEC) 513.

A radio section 515 amplifies power and converts frequency in order to communicate with a base station, which is included in a mobile communication system, via antenna 517. The power amplifier (PA) 519 and the transmitter/modulation circuitry are operationally responsive to the MCU 503, with an output from the PA 519 coupled to the duplexer 521 or circulator or antenna switch, as known in the art. The PA 519 also couples to a battery interface and power control unit 520.

In use, a user of mobile terminal 501 speaks into the microphone 511 and his or her voice along with any detected background noise is converted into an analog voltage. The analog voltage is then converted into a digital signal through the Analog to Digital Converter (ADC) 523. The control unit 503 routes the digital signal into the DSP 505 for processing therein, such as speech encoding, channel encoding, encrypting, and interleaving. In one embodiment, the processed voice signals are encoded, by units not separately shown, using a cellular transmission protocol such as enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., microwave access (WiMAX), Long Term Evolution (LTE) networks, code division multiple access (CDMA), wideband code division multiple access (WCDMA), wireless fidelity (WiFi), satellite, and the like, or any combination thereof.

The encoded signals are then routed to an equalizer 525 for compensation of any frequency-dependent impairments that occur during transmission though the air such as phase and amplitude distortion. After equalizing the bit stream, the modulator 527 combines the signal with a RF signal generated in the RF interface 529. The modulator 527 generates a sine wave by way of frequency or phase modulation. In order to prepare the signal for transmission, an up-converter 531 combines the sine wave output from the modulator 527 with another sine wave generated by a synthesizer 533 to achieve the desired frequency of transmission. The signal is then sent through a PA 519 to increase the signal to an appropriate power level. In practical systems, the PA 519 acts as a variable gain amplifier whose gain is controlled by the DSP 505 from information received from a network base station. The signal is then filtered within the duplexer 521 and optionally sent to an antenna coupler 535 to match impedances to provide maximum power transfer. Finally, the signal is transmitted via antenna 517 to a local base station. An automatic gain control (AGC) can be supplied to control the gain of the final stages of the receiver. The signals may be forwarded from there to a remote telephone which may be another cellular telephone, any other mobile phone or a land-line connected to a Public Switched Telephone Network (PSTN), or other telephony networks.

Voice signals transmitted to the mobile terminal 501 are received via antenna 517 and immediately amplified by a low noise amplifier (LNA) 537. A down-converter 539 lowers the carrier frequency while the demodulator 541 strips away the RF leaving only a digital bit stream. The signal then goes through the equalizer 525 and is processed by the DSP 505. A Digital to Analog Converter (DAC) 543 converts the signal and the resulting output is transmitted to the user through the speaker 545, all under control of a Main Control Unit (MCU) 503 which can be implemented as a Central Processing Unit (CPU) (not shown).

The MCU 503 receives various signals including input signals from the keyboard 547. The keyboard 547 and/or the MCU 503 in combination with other user input components (e.g., the microphone 511) comprise a user interface circuitry for managing user input. The MCU 503 runs a user interface software to facilitate user control of at least some functions of the mobile terminal 501 as described herein. The MCU 503 also delivers a display command and a switch command to the display 507 and to the speech output switching controller, respectively. Further, the MCU 503 exchanges information with the DSP 505 and can access an optionally incorporated SIM card 549 and a memory 551. In addition, the MCU 503 executes various control functions required of the terminal. The DSP 505 may, depending upon the implementation, perform any of a variety of conventional digital processing functions on the voice signals. Additionally, DSP 505 determines the background noise level of the local environment from the signals detected by microphone 511 and sets the gain of microphone 511 to a level selected to compensate for the natural tendency of the user of the mobile terminal 501.

The CODEC 513 includes the ADC 523 and DAC 543. The memory 551 stores various data including call incoming tone data and is capable of storing other data including music data received via, e.g., the global Internet. The software module could reside in RAM memory, flash memory, registers, or any other form of writable storage medium known in the art. The memory device 551 may be, but not limited to, a single memory, CD, DVD, ROM, RAM, EEPROM, optical storage, magnetic disk storage, flash memory storage, or any other non-volatile storage medium capable of storing digital data.

An optionally incorporated SIM card 549 carries, for instance, important information, such as the cellular phone number, the carrier supplying service, subscription details, and security information. The SIM card 549 serves primarily to identify the mobile terminal 501 on a radio network. The card 549 also contains a memory for storing a personal telephone number registry, text messages, and user specific mobile terminal settings.

In some embodiments, the mobile terminal 501 includes a digital camera comprising an array of optical detectors, such as charge coupled device (CCD) array 565. The output of the array is image data that is transferred to the MCU for further processing or storage in the memory 551 or both. In the illustrated embodiment, the light impinges on the optical array through a lens 563, such as a pin-hole lens or a material lens made of an optical grade glass or plastic material. In the illustrated embodiment, the mobile terminal 501 includes a light source 561, such as a LED to illuminate a subject for capture by the optical array, e.g., CCD 565. The light source is powered by the battery interface and power control module 520 and controlled by the MCU 503 based on instructions stored or loaded into the MCU 503.

As an example, an absorbent article may comprise a material (e.g., a nonwoven material) comprising a thermal weld with a tail extending from the thermal weld a distance of no greater than about 20mm, no greater than about 10 mm or no greater than about 5mm.

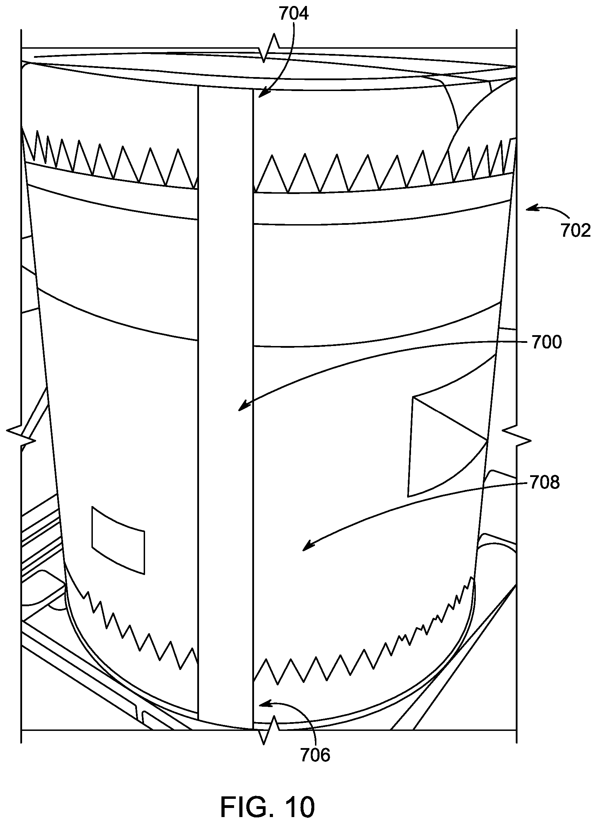

Referring to Fig. 10, Typically, rolls of webs are shipped from the roll goods manufacturer to absorbent article manufactures in a cylindrical bundle of 2-20 rolls, for example. The bundles may be about 1 meter to 2 meters in diameter, for example. The rolls are tightly wrapped with a plastic film, such as a polyethylene film, to protect the rolls from dirt and damage. Cardboard typically covers the bundle of the rolls on the flat ends of the cylindrical bundle to protect the ends. This plastic film needs to be removed prior to using the rolls in absorbent article manufacturing process. Typically, the film is manually cut off using a rotary sheer or cutter, however when cutting across the film over the curved outer surface of the rolls the manual rotary cutting may damage and cut into the outer revolutions of the web on the rolls, thereby ruining the outer revolutions of the web. The present inventors have discovered that by adding a sacrificial material 700 in a strip about the curved outer surface of the bundle 702 from the first end 704 to the second end 706 and under the film 708, automated cutting can occur to remove the film without damaging the outer revolutions of the rolls. In such a way, a robot may use a rotary sheer or cutter to cut into but not through the sacrificial material 700 while also cutting the film 708. Examples of the sacrificial material are heavy paper, cardboard, corrugated cardboard, polymer foam, and/or other low cost materials that provide sufficient caliper for protection of the underlying web. The sacrificial material may comprise a single layer or may be a laminate of multiple materials. The strip of sacrificial material may be from about 0.5 inches wide to about 15 inches wide, for example. This automated process significantly reduces scrap caused by the manual cutting and damage to the outer revolutions of the material.

### Examples/Combinations:

1. A method of providing a continuous web to an absorbent article manufacturing line, the method comprising:
   conveying a first portion of a first roll of material through a splice box and into the absorbent article manufacturing line including accumulating the first roll of material downstream of a splice location in the splice box;
   prior to expiration of the first roll of material, providing a second roll of the material extending into the splice box;
   using a vacuum to hold a second portion of the second roll of the material proximate to a leading edge thereof against a second component of a thermal welding apparatus;
   sensing upcoming expiration of the first roll of material;
   positioning a first component of the thermal welding apparatus proximate to the second component of the thermal welding apparatus;
   positioning the first portion of the first roll in an overlapping configuration with the second portion of the second roll;
   forming a thermal weld at the splice location between the first portion of the first roll of material and the second portion of the second roll of material using the thermal welding apparatus, wherein the first portion of the first roll comprises, in a cross-machine direction, a first side region and a second side region separated by a central region, wherein the second portion of the second roll comprises, in the cross-machine direction, a first side region and a second side region separated by a central region, and wherein the thermal weld is formed in the first side regions, the central regions, and the second side regions at the same time;
   cutting the first roll of material upstream of the thermal weld; and
   conveying the second roll of material through the splice box and into the absorbent article manufacturing line without stoppage of the absorbent article manufacturing line.
2. The method of Paragraph 1, comprising:
   extending the second roll of material into the splice box to a position downstream of the splice location;
   collecting a portion of the second roll of material downstream of the splice location to remove a plurality of outer layers of the second roll of material;
   cutting the collected material from a remainder of the second roll thereby forming the leading edge in the second roll of material; and
   retracting the leading edge of the second roll of material into the splice location to reduce or eliminate a first tail being formed in the continuous web.
3. The method of Paragraph 2, wherein the cutting the first roll of material upstream of the thermal weld step comprises cutting the first roll of material proximate to, but upstream of the thermal weld to reduce or eliminate a second tail being formed in the continuous web.
4. The method of any one of the preceding paragraphs, wherein the positioning the first component of the thermal welding apparatus proximate to the second component of the thermal welding apparatus comprises moving the first component and the second component toward each other.
5. The method of any one of the preceding paragraphs, wherein the thermal weld is not formed by the thermal welding apparatus traversing across the first material and the second material.
6. The method of any one of the preceding paragraphs, wherein the thermal welding apparatus comprises a source of vibration energy.
7. The method of any one of the preceding paragraphs, wherein the first component of the thermal welding apparatus is a sonotrode and the second component of the thermal welding apparatus is an anvil.
8. The method of any one of the preceding paragraphs, wherein the vacuum is a fluid pressure, and wherein a portion of the fluid pressure is provided through the source of vibration energy.
9. The method of any one of Paragraphs 1-5, wherein the thermal welding apparatus is one of a heat bar, a source of hot fluid, an ultrasonic welding apparatus or a mechanical welding apparatus.
10. The method of any one of the preceding paragraphs, wherein the first roll of the material and the second roll of material are the same material.
11. The method of any one of the preceding paragraphs, wherein the first roll of material or the second roll of material comprise a laminate of more than one material.
12. The method of any one of the preceding paragraphs, wherein the thermal weld is tapeless.
13. The method of any one of the preceding paragraphs, wherein the forming the thermal weld step comprises:
   a web stabilization step that takes an amount of time in a range from about 0.05 seconds to about 0.1 seconds;
   a welding step to form the thermal weld that takes an amount of time in the range of about 0.1 seconds to about 0.5 seconds; and
   a web cool-off step that takes an amount of time in a range from about 0.05 seconds to about 0.2 seconds.
14. The method of any one of the preceding paragraphs, wherein the second component of the thermal welding apparatus comprises an integrated vacuum bar with an embedded optical sensor, wherein the embedded optical sensor is configured to detect the leading edge of the second roll and wherein the integrated vacuum bar is configured to provide the vacuum to hold the second portion of the second roll against the component of the thermal welding apparatus.
15. The method of any one of the preceding paragraphs, comprising:
   using a clamping mechanism to hold the second portion of the second roll of the material proximate to the leading edge thereof against the second component of the thermal welding apparatus.
16. The method of any one of the preceding paragraphs, wherein the thermal welding apparatus comprises an integrated vacuum bar comprising:
   a plurality of web presence sensors configured to detect a presence of the second roll;
   wherein the using the vacuum to hold the second portion of the second roll of the material against the second component of the thermal welding apparatus is based on detection of the second roll by a first web presence sensor of the plurality of web presence sensors;
   wherein the method further comprises moving the second roll in an upstream direction after the second roll is held against the second component of the thermal welding apparatus, wherein the moving step is performed until a first web presence sensor of the plurality of web presence sensors no longer detect the presence of the second roll; and
   wherein the first web presence sensor is positioned between the second web presence sensor and the second component of the thermal welding apparatus.
17. The method of any one of the preceding paragraphs, wherein the splice box is automated.
18. The method of any one of the preceding paragraphs, wherein at least one of the first roll of the material and the second roll of the material are guided into and through the splice box by one or more robots.
19. The method of Paragraph 18, comprising:
   providing, at a leading edge of the second roll, a multi-zone sticker comprising a first zone that is attached to an outer layer of the second roll, a second zone that is attached along a leading edge between the outer layer and an inner layer of the second roll and a third zone that is not attached to the second roll;
   attaching, with a vacuum device, an arm of the one or more robots to the third zone of the multi-zone sticker; and
   moving the arm of the one or more robots with the attached third zone of the multi-zone sticker such that the second zone of the sticker is detached from the inner and outer layers along the leading edge of the second roll and the first zone remains attached to the outer layer of the second roll so that the leading edge of the second roll is moved by the arm of the one or more robots into the splice box.
20. The method of Paragraph 19, wherein the splice box includes a side facing an operator that defines one or more openings oriented along a movement axis of the one or more robots such that the moving step comprises moving the arm of the one or more robots with the attached third zone of the multi-zone sticker and the leading edge of the second roll along the movement axis of the one or more robots through one of the openings in the side of the splice box to a mandrel within the splice box.
21. The method of any one of the preceding paragraphs, wherein the first roll of material comprises a nonwoven material, a film, or a laminate comprising a nonwoven material and a film.
22. The method of any one of the preceding paragraphs, wherein the second roll of material comprises a nonwoven material, a film, or a laminate comprising a nonwoven material and a film.
23. The method of any one of Paragraphs 3-22, wherein the first tail has a length in the range of about 0.01mm to about 20mm.
24. The method of any one of Paragraphs 3-22, wherein the second tail has a length in the range of about 0.01mm to about 20mm.
25. A method of providing a continuous web to an absorbent article manufacturing line, the method comprising:
   conveying a first roll of material through a splice box and into the absorbent article manufacturing line;
   prior to expiration of the first roll of material, providing a second roll of the material extending into the splice box;
   extending the second roll of material into the splice box to a position downstream of a splice location;
   collecting a portion of the second roll of material downstream of the splice location to remove a plurality of outer layers of the second roll of material;
   cutting the collected material from a remainder of the second roll thereby forming the leading edge in the second roll of material;
   retracting the leading edge of the second roll of material into the splice location to reduce or eliminate a first tail being formed in the continuous web;
   positioning a first portion of the first roll of the material proximate to a tailing edge thereof adjacent a first component of a thermal welding apparatus at the splice location;
   using a vacuum to hold a second portion of the second roll of the material proximate to the leading edge thereof against a second component of the thermal welding apparatus at the splice location;
   positioning the first component of the thermal welding apparatus proximate to the second component of the thermal welding apparatus;
   positioning the first portion of the first roll in an overlapping configuration with the second portion of the second roll;
   forming a thermal weld at the splice location between the first portion of the first roll of material and the second portion of the second roll of material using the first and second components of the thermal welding apparatus, wherein the first portion of the first roll comprises, in a cross-machine direction, a first side region and a second side region separated by a central region, wherein the second portion of the second roll comprises, in the cross-machine direction, a first side region and a second side region separated by a central region, and wherein the thermal weld is formed in the first side regions, the central regions, and the second side regions at the same time;
   cutting the first roll of material upstream of the thermal weld; and
   conveying the second roll of material through the splice box and into the absorbent article manufacturing line without stoppage of the absorbent article manufacturing line.
26. The method of Paragraph 25, comprising:
   providing, at a leading edge of the second roll, a multi-zone sticker comprising a first zone that is attached to an outer layer of the second roll, a second zone that is attached along a leading edge between the outer layer and an inner layer of the second roll and a third zone that is not attached to the second roll;
   wherein the extending step comprises;
      attaching, with a vacuum device, an arm of the one or more robots to the third zone of the multi-zone sticker, and
      moving the arm of the one or more robots with the attached third zone of the multi-zone sticker such that the second zone of the sticker is detached from along the leading edge of the second roll and the first zone remains attached to the outer layer of the second roll as the leading edge of the second roll is moved by the arm of the one or more robots into the splice box to the position downstream of the splice location.
27. The method of Paragraph 26, wherein the splice box includes a side facing an operator that defines one or more openings oriented along a movement axis of the one or more robots such that the moving step comprises moving the arm of the one or more robots with the attached third zone of the multi-zone sticker and the leading edge of the second roll along the movement axis of the one or more robots through one of the openings in the side of the splice box to a mandrel within the splice box;
   and wherein the collecting step comprises winding the mandrel with the attached leading edge of the second roll until the plurality of outer layers are collected on the mandrel.
28. The method of Paragraph 27, wherein the one or more robots comprises the mandrel.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present disclosure have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of providing a continuous web to an absorbent article manufacturing line, the method comprising:
conveying a first portion of a first roll of material through a splice box and into the absorbent article manufacturing line including accumulating the first roll of material downstream of a splice location in the splice box;
prior to expiration of the first roll of material, providing a second roll of the material extending into the splice box;
using a vacuum to hold a second portion of the second roll of the material proximate to a leading edge thereof against a second component of a thermal welding apparatus;
sensing upcoming expiration of the first roll of material;
positioning a first component of the thermal welding apparatus proximate to the second component of the thermal welding apparatus;
positioning the first portion of the first roll in an overlapping configuration with the second portion of the second roll;
forming a thermal weld at the splice location between the first portion of the first roll of material and the second portion of the second roll of material using the thermal welding apparatus, wherein the first portion of the first roll comprises, in a cross-machine direction, a first side region and a second side region separated by a central region, wherein the second portion of the second roll comprises, in the cross-machine direction, a first side region and a second side region separated by a central region, and wherein the thermal weld is formed in the first side regions, the central regions, and the second side regions at the same time;
cutting the first roll of material upstream of the thermal weld; and
conveying the second roll of material through the splice box and into the absorbent article manufacturing line without stoppage of the absorbent article manufacturing line.

2. The method of Claim 1, comprising:
extending the second roll of material into the splice box to a position downstream of the splice location;
collecting a portion of the second roll of material downstream of the splice location to remove a plurality of outer layers of the second roll of material;
cutting the collected material from a remainder of the second roll thereby forming the leading edge in the second roll of material; and
retracting the leading edge of the second roll of material into the splice location to reduce or eliminate a first tail being formed in the continuous web.

3. The method of Claim 2, wherein the cutting the first roll of material upstream of the thermal weld step comprises cutting the first roll of material proximate to, but upstream of the thermal weld to reduce or eliminate a second tail being formed in the continuous web.

4. The method of any one of the preceding claims, wherein the positioning the first component of the thermal welding apparatus proximate to the second component of the thermal welding apparatus comprises moving the first component and the second component toward each other.

5. The method of any one of the preceding claims, wherein the thermal weld is not formed by the thermal welding apparatus traversing across the first material and the second material.

6. The method of any one of the preceding claims, wherein the thermal welding apparatus comprises a source of vibration energy.

7. The method of any one of the preceding claims, wherein the first component of the thermal welding apparatus is a sonotrode and the second component of the thermal welding apparatus is an anvil.

8. The method of any one of the preceding claims, wherein the vacuum is a fluid pressure, and wherein a portion of the fluid pressure is provided through the source of vibration energy.

9. The method of any one of Claims 1-5, wherein the thermal welding apparatus is one of a heat bar, a source of hot fluid, an ultrasonic welding apparatus or a mechanical welding apparatus.

10. The method of any one of the preceding claims, wherein the first roll of the material and the second roll of material are the same material.

11. The method of any one of the preceding claims, wherein the first roll of material or the second roll of material comprise a laminate of more than one material.

12. The method of any one of the preceding claims, wherein the thermal weld is tapeless.

13. The method of any one of the preceding claims, wherein the forming the thermal weld step comprises:
a web stabilization step that takes an amount of time in a range from about 0.05 seconds to about 0.1 seconds;
a welding step to form the thermal weld that takes an amount of time in the range of about 0.1 seconds to about 0.5 seconds; and
a web cool-off step that takes an amount of time in a range from about 0.05 seconds to about 0.2 seconds.

14. The method of any one of the preceding claims, wherein the second component of the thermal welding apparatus comprises an integrated vacuum bar with an embedded optical sensor, wherein the embedded optical sensor is configured to detect the leading edge of the second roll and wherein the integrated vacuum bar is configured to provide the vacuum to hold the second portion of the second roll against the component of the thermal welding apparatus.

15. The method of any one of the preceding claims, comprising:
using a clamping mechanism to hold the second portion of the second roll of the material proximate to the leading edge thereof against the second component of the thermal welding apparatus.
